(19) **Europäisches Patentamt / European Patent Office / Office européen des brevets**

(11) **EP 4 693 333 A1**

(12) **EUROPEAN PATENT APPLICATION**

(43) Date of publication:
11.02.2026 Bulletin 2026/07

(21) Application number: 25168966.7

(22) Date of filing: 07.04.2025

(51) International Patent Classification (IPC):
$G16H\ 50/20^{(2018.01)}$    $G16H\ 20/30^{(2018.01)}$
$G16H\ 30/40^{(2018.01)}$    $G16H\ 50/70^{(2018.01)}$
$A61B\ 5/00^{(2006.01)}$    $A61B\ 3/113^{(2006.01)}$
$A61B\ 5/11^{(2006.01)}$

(52) Cooperative Patent Classification (CPC):
G16H 20/30; A61B 3/113; A61B 5/4023;
G16H 30/40; G16H 50/20; G16H 50/70;
A61B 5/1128

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 05.08.2024 KR 20240104114
05.08.2024 KR 20240104115

(71) Applicants:
• NeuroEars Co., Ltd.
Chuncheon-si, Gangwon-do 24232 (KR)
• Industry Academic Cooperation Foundation,
Hallym University
Chuncheon-si, Gangwon-do 24252 (KR)

(72) Inventors:
• HONG, Sung Kwang
13600 Seongnam-si (KR)
• KWON, Ha Neul
14081 Anyang-si (KR)
• YOON, Dae Hee
15840 Gunpo-si (KR)
• LIM, Eun Cheon
08746 Seoul (KR)

(74) Representative: IPAZ
Bâtiment Platon
Parc Les Algorithmes
91190 Saint-Aubin (FR)

(54) **BALANCE FUNCTION MANAGEMENT SYSTEM AND METHOD FOR GENERATING INFORMATION ON BALANCE FUNCTION STATUS AND PERFORMING BALANCE FUNCTION REHABILITATION PROGRAM BY TRACKING EYE AND HEAD POSITION CHANGES IN VIDEOS, RECORDING MEDIUM STORING PROGRAM FOR EXECUTING THE SAME, AND RECORDING MEDIUM STORING PROGRAM FOR EXECUTING THE SAME**

(57) A balance function management system includes at least one processor, and a memory that stores instructions executable by the processor and stores at least one artificial neural network model executed on a computing device, in which the at least one processor may input frame images of n videos of a subject captured by n (natural number) cameras to at least one artificial neural network model to acquire at least one of information related to head coordinates, coordinates of a pupil center, and eye phase changes of the subject according to an order of frame images of an m-th (natural number from 1 to n) video, and use the information to generate information on a balance function status or information related to head movement and eye movement for performing a balance function rehabilitation program.

FIG. 1

**Description**

**CROSS-REFERENCE TO RELATED APPLICATIONS**

This application claims the priority of Korean Patent Applications No. 10-2024-0104114 and 10-2024-0104115 filed on August 5, 2024, in the Korean Intellectual Property Office, the disclosure of which is incorporated herein by reference.

**BACKGROUND**

**Field**

The present disclosure relates to a balance function management system and method for generating information on a balance function status and a balance function rehabilitation program, and more particularly, to a balance function management system and method for generating information on a balance function status of a subject by tracking eye and head position changes of the subject in a video of the subject captured by at least one camera and performing a balance function rehabilitation program of the subject, a recording medium storing a program for executing the same, and a computer program stored in the recording medium.

**Description of the Related Art**

The contents described in this section merely provide background information for an exemplary embodiment described in the present specification and do not necessarily constitute the related art.

Although a nystagmus test, which evaluates the vestibulo-ocular reflex when the dizziness occurs, has been proven to be cost-effective and superior in diagnostic sensitivity to MRI, it has the problem of difficulty in conducting the test and interpretation. Therefore, there is no device for conducting the nystagmus test. As a result, the nystagmus test is not well utilized actually in emergency rooms or primary hospitals where dizziness patients visit the most, and thus, the dizziness is often diagnosed by relying on unnecessary imaging tests or blood tests. This causes waste of medical expenses in terms of cost effectiveness, and often leads to poor prognosis for patients due to lack of proper diagnosis. In particular, it is difficult for patients to perform vestibular rehabilitation treatment on their own to recover from acute dizziness symptoms, which delays recovery from the dizziness and causes a decrease in quality of life.

[Related Art Document]

[Patent Document]

Korean Patent Laid-Open Publication No. 10-2004-0107677 (December 23, 2004)

**SUMMARY**

An object to be achieved by the present specification is to provide a balance function management system for generating information on a balance function status and a balance function rehabilitation program.

The present specification is not limited to the above-described problems, and other problems that are not described may be obviously understood by those skilled in the art from the following description.

A balance function management system according to an exemplary embodiment of the present specification may include: at least one processor; and a memory that stores instructions executable by the processor and stores at least one artificial neural network model executed on a computing device, in which the at least one processor may input frame images of n videos of a subject captured by n (natural number) cameras to at least one artificial neural network model to acquire at least one of information related to head coordinates, coordinates of a pupil center, and eye phase changes of the subject according to an order of frame images of an m-th (natural number from 1 to n) video, and use the information to generate information related to head movement and eye movement for generating information on a balance function status or performing a balance function rehabilitation program.

The at least one processor may include: a head coordinate acquirer that executes a first artificial neural network model stored in the memory, and inputs frame images of the m-th video or multi-frame images concatenating frame images of n videos to the first artificial neural network model to acquire the information related to the head coordinates according to the m-th video; an eye coordinate acquirer that executes a second artificial neural network model stored in the memory, and inputs the information related to the head coordinates to the second artificial neural network model to acquire information related to coordinates of a pupil center according to the m-th video; and a phase change acquirer that executes a third artificial neural network model stored in the memory, and inputs information related to coordinates of a pupil center according to a time sequence of the multi-frame image or the frame image of the m-th video to the third artificial neural network model to acquire the information related to the eye phase changes according to the m-th video.

The first artificial neural network model may be an artificial neural network model trained by allowing the at least one processor to use, as training data, facial feature points extracted from frame images of at least one video in which a human face is captured or multi-frame images in which frame images of multiple videos in which a human face is captured are concatenated and coordinates of the feature points; wherein the feature point is a feature point positioned within a preset area in the frame images or the multi-frame images.

The second artificial neural network model may be an artificial neural network model trained to generate information related to coordinates of a pupil center by allowing the at least one processor to use, as training data, eye area images extracted to include an eye from frame images of at least one video in which a human face is captured or multi-frame images in which frame images of multiple videos in which a human face is captured are concatenated.

The at least one processor may generate pupil area images in which an area occupied by the pupil and the remaining area have different pixel values in the eye area images, and train the second artificial neural network model using the pupil area images or an array of pixel values of the pupil area images as the training data.

The at least one processor may train the second artificial neural network model to generate eye feature points and coordinate information of the feature points from the eye area images, and to generate horizontal coordinate values and vertical coordinate values of the pupil center using coordinates of a plurality of preset feature points.

The memory may store data of at least one virtual object, and the second artificial neural network model may be an artificial neural network model trained to generate information related to coordinates of a pupil center by allowing the at least one processor to use training data that includes a parameter value that changes at least one of parameters related to head rotation, eye rotation, and camera settings of the virtual object and an image of the virtual object acquired according to the parameter value.

The third artificial neural network model may be an artificial neural network model trained to generate an eye rotation value by allowing the at least one processor to use, as training data, information related to eye phase changes generated according to a time sequence of an eye area image extracted to include an eye from frame images of at least one video in which a human face is captured or multi-frame images in which frame images of multiple videos in which a human face is captured are concatenated.

The third artificial neural network model may be an artificial neural network model trained by allowing the at least one processor to use information comparing pixel values of an area occupied by an iris between eye area images corresponding to each frame image of each video, or to each frame image of each video in the multi-frame images.

The at least one processor may calculate a size of an area occupied by a pupil in the eye area images and adjust a size of a target eye area image using a size of an area occupied by a pupil in a preceding eye area image.

The at least one processor may include: a head movement generator that generates information related to head movement in an m-th (natural number from 1 to n) video using the information related to the head coordinates generated from at least one artificial neural network model; an eye movement generator that generates information related to eye movement in the m-th video using information related to coordinates of a pupil center or eye phase changes generated from at least one artificial neural network model; a speed information generator that generates information related to head and eye movement speeds in the m-th video using the information related to the head movement and the eye movement; and a balance function status information generator that generates information on a balance function status of the subject using the information related to the head and eye movement speeds.

The speed information generator may generate the information related to the head movement speed and the eye movement speed within a preset time based on a time when the head movement becomes greater than or equal to a preset threshold value.

The balance function status information generator may calculate a gain using a time value at which the head movement speed is maximum and a time value at which the eye movement speed is maximum.

When n is greater than or equal to 2, the head movement generator may further generate reference head movement information by calculating statistical values of the information related to the head coordinates according to the m-th video, and the eye movement generator may further generate reference eye movement information by calculating statistical values of the information related to the coordinates of the pupil center and the eye phase changes according to the m-th video.

The at least one processor may include: a head movement generator that generates the information related to head movement in the m-th (natural number from 1 to n) video using the information related to the head coordinates acquired from at least one artificial neural network model; an eye movement generator that generates the eye movement information in the m-th video using the information related to the coordinates of a pupil center or the eye phase changes acquired from the at least one artificial neural network model; a target output generator that outputs a virtual target to a display device; a head direction provider that provides direction information on the head movement to the subject; and a feedback provider that provides feedback according to the head movement and the eye movement of the subject.

Other detailed contents of the present disclosure are described in a detailed description and are illustrated in the drawings.

[Effect of the Invention]

According to one aspect of the present specification, the balance function management system can input the video in which the subject performing the balance function status check is captured using the smart phone, the webcam, etc., to the artificial neural network model to calculate the eye movement and head movement without separate medical equipment and generate the information on the balance function status.

According to another aspect of the present specification, the balance function management system can be utilized as the clinical decision support system for the dizzy patient that generates the information on the balance function status.

According to another aspect of the present specification, the balance function management system can provide the important information for quickly distinguishing the peripheral and central dizziness with only the single smart phone without using the separate medical device in the emergency room environment or without the patient wearing the separate device.

According to another aspect of the present specification, the balance function management system can provide the remote balance function rehabilitation program by outputting the movement information on patient's head position change and eye movement to the display device to enable more accurate balance rehabilitation.

Effects of the present disclosure are not limited to the effects described above, and other effects that are not mentioned may be obviously understood by those skilled in the art from the following description.

The effects of the present disclosure are not limited to the aforementioned effects, and other effects, which are not mentioned above, will be apparently understood to a person having ordinary skill in the art from the following description.

The objects to be achieved by the present disclosure, the means for achieving the objects, and the effects of the present disclosure described above do not specify essential features of the claims, and, thus, the scope of the claims is not limited to the disclosure of the present disclosure.

**BRIEF DESCRIPTION OF DRAWINGS**

The above and other aspects, features and other advantages of the present disclosure will be more clearly understood from the following detailed description taken in conjunction with the accompanying drawings, in which:

FIG. 1 is a reference diagram illustrating a scene of capturing a subject with at least one camera;

FIG. 2 is a reference diagram illustrating a scene of a subject captured by a camera included in a terminal;

FIG. 3 is a block diagram of a balance function management system according to a first exemplary embodiment of the present specification;

FIG. 4 is an example of a scene of capturing a subject that performs a balance function status check and/or a balance function rehabilitation program according to an exemplary embodiment of the present specification;

FIG. 5 is a diagram illustrating an example of a scene of capturing a subject that performs a balance function status check and/or a balance function rehabilitation program according to another exemplary embodiment of the present specification;

FIG. 6 is a diagram illustrating an example of preprocessing an eye area image according to an exemplary embodiment of the present specification;

FIG. 7 is a diagram illustrating an example of generating training data of a second ANN model according to another exemplary embodiment of the present specification;

FIG. 8 is a block diagram of a balance function management system for generating information on a balance function status according to an exemplary embodiment of the present specification;

FIG. 9 is a diagram illustrating an example of outputting information on a balance function status to a display according to an exemplary embodiment of the present specification;

FIG. 10 is a block diagram of a balance function management system for performing a balance function rehabilitation program according to an exemplary embodiment of the present specification;

FIG. 11 is a diagram illustrating an example of a scene performing the balance function rehabilitation program according to an exemplary embodiment of the present specification;

FIG. 12 is a block diagram of a balance function management system for generating information on a balance function status and performing a balance function rehabilitation program according to an exemplary embodiment of the present specification;

FIG. 13 is a block diagram of a balance function management system according to a second exemplary embodiment of the present specification;

FIG. 14 is a reference diagram illustrating an example in which a head coordinate learning unit concatenates frame images according to an exemplary embodiment of the present specification;

FIG. 15 is a diagram illustrating an example of a multi-frame image of a scene where a subject performing a balance function status check and/or a balance function rehabilitation program according to an exemplary embodiment of the present specification is captured;

FIG. 16 is a diagram illustrating an example of the multi-frame image of the scene where the subject performing the balance function status check and/or the balance function rehabilitation program according to an exemplary embodiment of the present specification is captured;

FIG. 17 is a diagram illustrating an example of preprocessing an eye area image according to an exemplary embodiment of the present specification;

FIG. 18 is a block diagram of a balance function management system for generating information on a balance function status according to an exemplary embodiment of the present specification;

FIG. 19 is a block diagram of a balance function management system for performing a balance function rehabilitation program according to an exemplary embodiment of the present specification;

FIG. 20 is a block diagram of a balance function management system for generating information on a balance function status and performing a balance function rehabilitation program according to an exemplary embodiment of the present specification;

FIG. 21 is a flowchart of a method of generating information on a balance function status according to an exemplary embodiment of the present specification; and

FIG. 22 is a flowchart of a balance function rehabilitation method according to an exemplary embodiment of the present specification.

## DETAILED DESCRIPTION OF THE EMBODIMENT

Hereinafter, exemplary embodiments of the present disclosure will be described in detail with reference to the accompanying drawings.

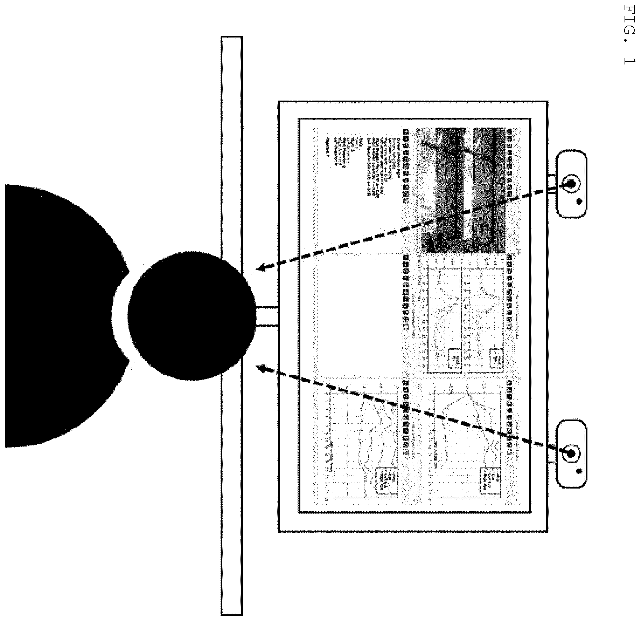

FIG. 1 is a reference diagram illustrating a scene of capturing a subject with at least one camera.

Referring to FIG. 1, a balance function management system according to an exemplary embodiment of the present specification may generate head movement and eye movement information of a subject using n videos of the subject captured by n (a natural number) cameras. The n cameras may be installed at preset positions to capture a face of a subject. The n cameras may capture a subject at different angles.

The balance function management system may receive video data of the subject captured by the n cameras. The balance function management system may display the received video data on a display device. The balance function management system may display m-th videos of a subject captured by an m-th (natural number from 1 to n) camera on the display device, respectively. Hereinafter, the m-th video may refer to all videos from a first video to an n-th video.

The balance function management system may generate information related to head and eye movement speeds using the information related to the head and eye movements, and display the generated information on the display device.

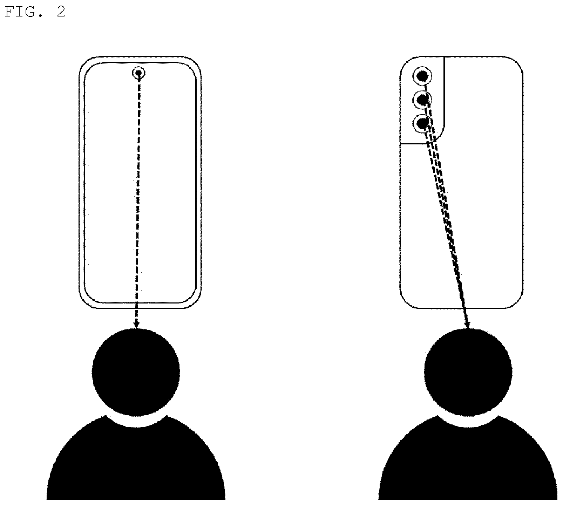

FIG. 2 is a reference diagram illustrating a scene of a subject captured by a camera included in a terminal.

Referring to FIG. 2, according to another exemplary embodiment of the present specification, the balance function management system may generate information related to head and eye movements using a video captured by a front camera (left of FIG. 2) and/or a rear camera (right of FIG. 2) of a terminal such as a smart phone or a tablet computer. When capturing the subject using the rear camera of the terminal, the balance function management system may display each video captured by at least one camera included in the rear of the terminal on the display device.

The subject may be captured by the camera included in the computing device and/or the camera connected to the computing device in a wired and/or wireless manner. Various types of cameras such as a webcam and an action camera may be used as the camera, etc.

The balance function management system may generate information related to the balance function status of the subject by using n videos of the subject performing a video head impulse test, a spontaneous nystagmus test, a saccade test, etc., which corresponds to an example, and the present disclosure is not limited to the tests.

Hereinafter, a balance function management system according to a first exemplary embodiment of the present specification will be described.

The balance function management system according to the first exemplary embodiment of the present specification may include at least one processor and a memory that stores instructions executable by the processor and stores at least one artificial neural network model (ANN model) executed on a computing device.

The balance function management system may allow the at least one processor to input frame images of n videos of the subject captured by n cameras to at least one ANN model, and acquire at least one of information related to head coordinates, coordinates of pupil center, and eye phase changes of the subject according to the order of the frame images. The at least one processor may use the information to generate head movement information and eye movement information.

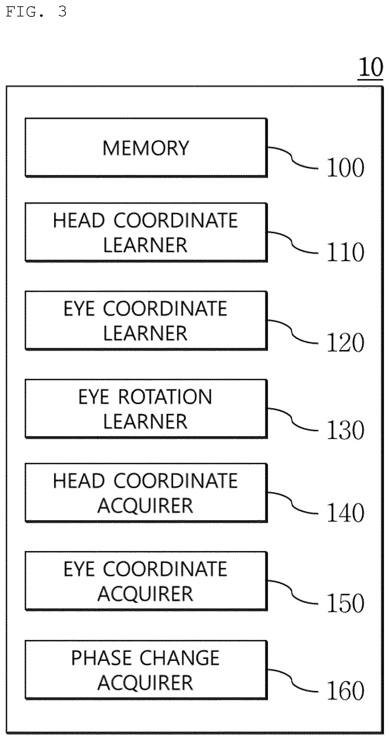

FIG. 3 is a block diagram of the balance function management system according to the first exemplary embodiment of the present specification.

Referring to FIG. 3, a balance function management system 10 according to the first exemplary embodiment of the present specification may include a memory 100, a head coordinate learner 110, an eye coordinate learner 120, an eye rotation learner 130, a head coordinate acquirer 140, an eye coordinate acquirer 150, and a phase change acquirer 160.

The memory 100 may store at least one of a first ANN model that generates information related to head coordinates, a second ANN model that acquires information related to coordinates of a pupil center, and a third ANN model that acquires information related to eye phase changes.

According to an exemplary embodiment of the present specification, the first ANN model may be trained by allowing the head coordinate learner 110 to use facial feature points and coordinates of the feature points extracted from at least one frame image of a video in which a human face is captured as training data.

For example, the video in which the human face is captured may be captured by a single camera. The video in which the human face is captured may be a video in which a scene of the subject performing a video head impulse test, a spontaneous nystagmus test, a saccade test, etc.

The head coordinate learner 110 may extract feature points for a human face from each frame image of the video in which the human face is captured. For example, the feature points may include feature points for a tip of a nose, a left outer canthus, a right outer canthus, and a forehead of a person. The positions of the feature points may not change even if a person blinks. Since the technology for generating feature points and feature point coordinates from a human head is a technology widely known to those skilled in the art, a detailed description thereof will be omitted.

The head coordinate learner 110 may generate coordinate information of feature points extracted from each frame image. The head coordinate learner 110 may train a first ANN model to generate three-dimensional head coordinates for each frame image using the feature points and coordinates of the feature points extracted from each frame image as training data. The 3D head coordinates may refer to 3D coordinates of a head according to a 3D standard head model. The 3D coordinates of the head may include coordinates for all feature points of the head. The 3D coordinates of the head may include contents related to an index number for each feature point. In this case, the head coordinate learner 110 may train the first ANN model to generate the index number based on the feature points of the tip of the nose. In addition, the head coordinate learner 110 may also be trained to further generate head movement information using 3D coordinates extracted from each frame image.

In addition, the head coordinate learner 110 may train the first ANN model further using, as training data, data on a 3D standard head model, such as the frame image from which the feature points are extracted, a 3D morphable model (3DMM), a faces learned with an articulated model and expressions (FLAME) model.

The head coordinate learner 110 may extract feature points after adjusting the sync of the multiple videos when extracting the feature points from the multiple videos in which a person is captured using a plurality of cameras. The head coordinate learner 110 may adjust the sync of the multiple videos using an algorithm such as a specific audio signal of the multiple videos and feature point matching-based synchronization.

According to an exemplary embodiment of the present specification, the head coordinate learner 110 may use feature points having coordinate values according to preset criteria as training data. The head coordinate learner 110 may select the feature points having the coordinate values according to the preset criteria as training data.

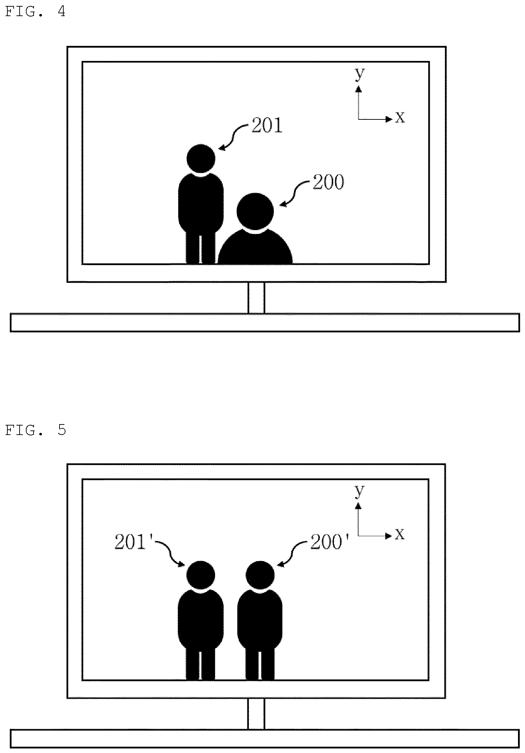

FIG. 4 is an example of a scene of capturing a subject that performs a balance function status check and/or a balance function rehabilitation program according to an exemplary embodiment of the present specification.

Referring to FIG. 4, the video in which the person is captured may be a video of performing the balance function status check and/or the balance function rehabilitation program using the balance function management system 10.

When performing the balance function status check, the subject 200 may be sitting on a chair and the examiner 201 may be standing behind the subject.

The feature points extracted from the face of the subject 200 may be positioned relatively lower than the feature points extracted from the face of the examiner 201. This may refer to that a y-coordinate value of the feature points extracted from the face of the subject 200 is relatively smaller than a y-coordinate value of the feature points extracted from the face of the examiner 201.

The head coordinate learner 110 may extract, as training data, feature points with relatively smaller y-coordinate values among feature points of the same portion extracted from the faces of the subject 200 and the examiner 201. The head coordinate learner 110 may extract feature points positioned in an area below the preset y-coordinate value as training data.

In addition, the head coordinate learner 110 may cluster feature points extracted from the faces of the subject 200 and the examiner 201. Thereafter, the head coordinate learner 110 may extract a set of feature points positioned at a relatively lower side as training data.

FIG. 5 is a diagram illustrating an example of a scene of capturing a subject that performs a balance function status check and/or a balance function rehabilitation program according to another exemplary embodiment of the present specification.

Referring to FIG. 5, in the video, the balance function status check and/or the balance function rehabilitation program may be performed while both the subject 200' and examiner 201' are standing. In this case, the feature points extracted from the face of the subject 200' may be relatively closer to the center of the display screen than the feature points extracted from the face of the examiner 201'. The head coordinate learner 110 may extract the feature points having the coordinate values that are relatively closer to the center of the display screen as training data.

In the video, when the head of the subject quickly rotates, the face of the subject may not be clearly captured in the frame image. In this case, the head coordinate learner 110 may not extract the feature points from the face of the subject.

The head coordinate learner 110 may track the coordinates of the feature points extracted from the frame image preceding an arbitrary frame image. When the feature points are not extracted from the face of the subject in the arbitrary frame image, the head coordinate learner 110 may extract the training data using the image of the subject in the preceding frame image.

According to an exemplary embodiment of the present specification, the eye coordinate learner 120 may extract an eye area image of a subject, which is an image of an area including an eye of the subject, from the video. The eye coordinate learner 120 may train the second ANN model to generate the information related to the coordinates of the pupil center by using the eye area image of the subject extracted from each frame image as training data.

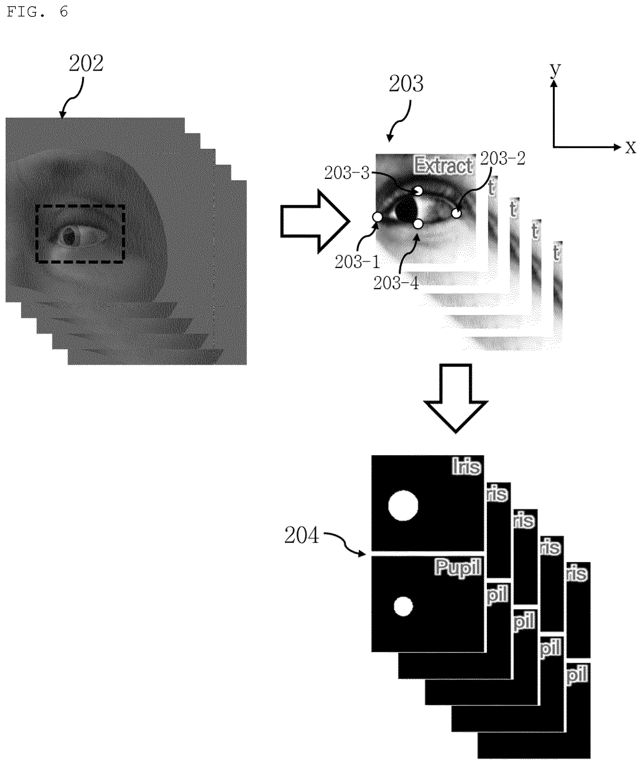

FIG. 6 is a diagram illustrating an example of preprocessing an eye area image according to an exemplary embodiment of the present specification.

Referring to FIG. 6, the eye coordinate learner 120 may extract an eye area image 203 according to each frame image 202. The eye coordinate learner 120 may extract an image inside a bounding box of an eye area from each frame image 202 as the eye area image 203. The eye coordinate learner 120 may segment the iris and pupil areas from the eye area image 203. Since the technology of extracting the eye area from the human face and segmenting the iris and pupil areas is a technology widely known to those skilled in the art, a detailed description thereof will be omitted.

The eye coordinate learner 120 may estimate an area for a part where the iris and/or pupil are covered by an eyelid. The eye coordinate learner 120 may estimate the covered part using an ellipse fitting algorithm, a circle Hough transform algorithm, or the like. Alternatively, the eye coordinate learner 120 may segment the iris and pupil areas using the ANN model that has been previously trained to segment the iris and pupil areas.

The eye coordinate learner 120 may generate a mask image in which the area occupied by the iris and/or pupil and the remaining area have different pixel values in the eye area image 203. The mask image may be displayed in white or black for the area occupied by the iris and/or pupil, and displayed in black or white for the remaining area.

The eye coordinate learner 120 may train the second ANN model to generate the information related to the coordinates of the pupil center using the mask image 204 and/or two-dimensional pixel values of the mask image 204 as training data.

As another example, the eye coordinate learner 120 may train the second ANN model using a heatmap model that segments and displays the iris and/or pupil area in the eye area image 203.

According to an exemplary embodiment of the present specification, the eye coordinate learner 120 may train the second ANN model to generate the eye feature points and coordinate information of the feature points from the eye area image 203, and to generate the horizontal coordinate values and vertical coordinate values of the pupil center using the coordinates of the plurality of preset feature points. The eye coordinate learner 120 may extract normalized coordinates of a pupil center using the coordinates of the plurality of feature points extracted from the eye area image 203.

For example, the eye coordinate learner 120 may use a line segment connecting the feature point 203-1 for the medial canthus and the feature point 203-2 for the outer canthus as a horizontal axis for the coordinates of the pupil center. The x-coordinate of the feature point 203-1 for the medial canthus and the x-coordinate of the feature point 203-2 for the outer canthus may correspond to both extreme values of the horizontal axis. The eye coordinate learner 120 may calculate the horizontal coordinates of the normalized pupil center using the horizontal coordinate values of the pupil center compared to the length of the entire horizontal axis.

In addition, the eye coordinate learner 120 may extract vertical coordinates of the normalized pupil center using feature points related to upper and lower eyelids among the feature points extracted from the eye area image 203. In this case, an upper eyelid feature point 203-3 with the largest y-axis coordinate among the feature points extracted from the eye may be used. A lower eyelid feature point 203-4 with the smallest y-axis coordinate among the feature points extracted from the eye may be used.

The eye coordinate learner 120 may use a line segment connecting the upper eyelid feature point 203-3 and the lower eyelid feature point 203-4 as a vertical axis for the coordinates of the pupil center. The y-coordinate of the upper eyelid feature point 203-3 and the y-coordinate of the lower eyelid feature point 203-4 may correspond to the two extreme values of the vertical axis. The eye coordinate learner 120 may calculate the horizontal coordinates of the normalized pupil center using the vertical coordinate values of the pupil center compared to the length of the entire vertical axis.

In the case of the eye area image rotating 30° clockwise based on the eye area image 203, the feature point 203-1 for the medial canthus and the feature point 203-2 for the outer canthus may not be positioned on the same horizontal line, and the upper eyelid feature point 203-3 and the lower eyelid feature point 203-4 may not be positioned on the same vertical line. In this case, the eye coordinate learner 120 may transform the image so that in the rotating eye area image, the feature point 203-1 for the medial canthus and the feature point 203-2 for the outer canthus are positioned on the same horizontal line, and the upper eyelid feature point 203-3 and the lower eyelid feature point 203-4 are positioned on the same vertical line. In this case, the eye coordinate learner 120 may transform the rotating eye area image using an affine transform, etc.

The eye coordinate learner 120 may train the second ANN model further using the horizontal coordinate values and the vertical coordinate values of the generated normalized pupil center as training data.

In addition, the eye coordinate learner 120 may calculate the average value of the horizontal coordinate values and the vertical coordinate values of the pupil center calculated from the multiple frame images whose synchronization matches each other in the multiple videos. The eye coordinate learner 120 may train the second ANN model further using the average value of the horizontal coordinate values and the vertical coordinate values of the pupil center as training data. The information related to the coordinates of the pupil center may include the vertical coordinate values and the horizontal coordinate values of the pupil center, the movement information of the pupil center in the vertical direction and the movement information of the pupil center in the horizontal direction, etc., according to each frame image. The information related to the coordinates of the pupil center may include contents about the two-dimensional coordinates.

According to another exemplary embodiment of the present specification, the memory 100 may store data of at least one virtual object. The second ANN model may be trained to generate information related to the coordinates of the pupil center by allowing the eye coordinate learner 120 to use training data that includes a parameter value obtained by changing at least one of parameters related to head rotation, eye rotation, and camera settings of a parameter of a virtual object and an image of the virtual object acquired according to the parameter value.

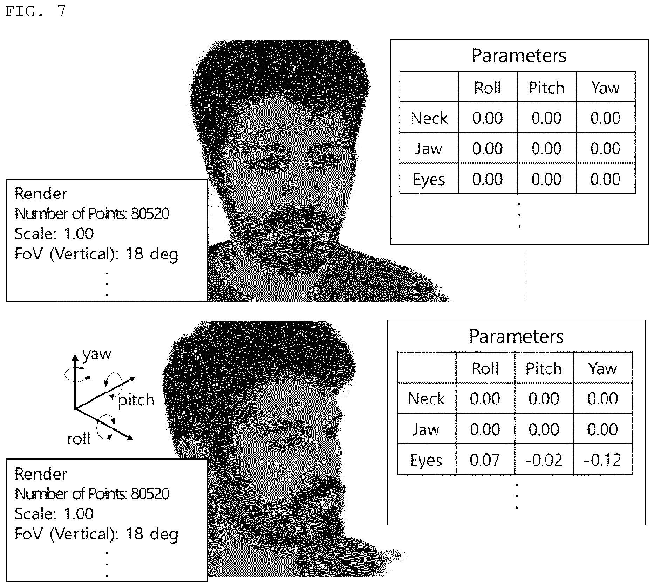

FIG. 7 is a diagram illustrating an example of generating training data of a second ANN model according to another exemplary embodiment of the present specification.

Referring to FIG. 7, the eye coordinate learner 120 may change at least one of the parameters related to the head rotation, the eye rotation, and the camera settings of the virtual object. As an example, the eye coordinate learner 120 may set parameter values so that the virtual camera captures the virtual object from the front (upper drawing of FIG. 7). The eye coordinate learner 120 may change parameter values so that the virtual camera captures the virtual object from the right (lower drawing of FIG. 7). In addition, the eye coordinate learner 120 may change parameter values for a distance between the virtual camera and the virtual object.

The eye coordinate learner 120 may set parameter values so that the head and/or the eye of the virtual object rotates in at least one direction of a roll, a pitch, and a yaw.

The eye coordinate learner 120 may change at least one of the parameters and acquire the image of the virtual object. The eye coordinate learner 120 may train the second ANN model using the parameter values and the virtual object according to the parameter values. In this case, the second ANN model may be trained to generate the information on the two-dimensional coordinates and/or three-dimensional coordinates of the pupil center.

The virtual object may mean a Gaussian avatar generated using a 3D Gaussian splatter. The eye coordinate learner 120 may control a latent vector of the virtual object to change Euler coordinates of the head and pupil of the virtual object.

In addition, the memory 100 may store labeling data including at least one of head coordinates, coordinates of a pupil center, and information related to camera settings according to an image in which a human face is captured. The eye coordinate learner 120 may train the second ANN model using the data.

In addition, the eye coordinate learner 120 may train the second ANN model using at least one of training data using the eye area image, training data obtained according to parameter changes of the virtual object, and labeling data.

According to an exemplary embodiment of the present specification, the eye rotation learner 130 may extract an eye area image, which is an image of an area including an eye extracted from each frame image of a video in which a human face is captured. The eye rotation learner 130 may train the third ANN model to generate an eye rotation value using training data including information related to the eye phase changes generated according to the time sequence of the eye area image.

The eye rotation learner 130 may compare an eye area image extracted from an arbitrary frame image with eye area images extracted from frame images within a preset time range based on the corresponding frame image to generate the information related to the eye phase changes. For example, the eye rotation learner 130 may compare an eye area image extracted from an arbitrary frame image with eye area images extracted from frame images acquired within 0.1 seconds or so based on the corresponding frame image to generate the information related to the eye phase changes.

According to an exemplary embodiment of the present specification, the eye rotation learner 130 may extract an iris area image, which is an image of an area occupied by an iris, from the eye area image. The eye rotation learner 130 may train the third ANN model using information related to a phase change of the iris according to the time sequence of the iris area image.

More specifically, the eye rotation learner 130 may compare an iris area image extracted from an arbitrary frame image with an iris area image extracted from frame images within a preset time range based on an arbitrary frame image. The iris area image may be a mask image in which an area occupied by an iris is distinguished by different pixel values from other areas.

In addition, the eye rotation learner 130 may generate the information related to the phase change using the mask image of the iris generated by the eye coordinate learner 120.

The eye rotation learner 130 may generate the information related to the phase change of the iris by comparing the pixel values of the iris area image extracted from the arbitrary frame image with those of other iris area images. The eye rotation learner 130 may generate phase cross correlation values for pixel values of the iris area image extracted from the arbitrary frame image and other iris area images by using phase cross correlation analysis. The information related to the phase change calculated by using the phase cross correlation analysis may include contents about the change in the angle of the iris.

The eye rotation learner 130 may generate the phase cross correlation value by a method of obtaining a cross correlation value upsampled by a fast Fourier transform (FFT). The eye rotation learner 130 may calculate an initial estimate value of a cross correlation peak using the FFT, and then generate the phase cross correlation value by precisely estimating a phase shift of the upsampled signal using the discrete Fourier transform (DFT) in a preset area based on the estimated value.

The eye rotation learner 130 may train the third ANN model using the image and the phase cross correlation value of the iris area as the training data.

According to an exemplary embodiment of the present specification, the eye rotation learner 130 may calculate the size of the area occupied by the pupil in the eye area image extracted from the frame image of the m-th video. The eye rotation learner 130 may adjust the size of the target eye area image according to the preset criteria. The target eye area image refers to an eye area image extracted from the arbitrary frame image.

The eye rotation learner 130 may compare the sizes of the areas occupied by the pupils calculated from the target eye area image extracted from the arbitrary frame image and the preceding eye area image extracted from the immediately preceding frame image. The eye rotation learner 130 may adjust the size of the target eye area image so that the size of the area occupied by the pupil extracted from the target eye area image has a value within a preset difference value from the size of the area occupied by the pupil extracted from the preceding eye area image. The eye rotation learner 130 may calculate the phase cross correlation value after adjusting the sizes of each eye area image.

In addition, the eye rotation learner 130 may generate a bounding box of an area including an eye in the frame image. The eye rotation learner 130 may extract the pupil center within the bounding box. Alternatively, the eye rotation learner 130 may receive the information related to the coordinates of the pupil center generated from the second ANN model.

The eye rotation learner 130 may adjust the bounding box so that the pupil center is positioned at the center of the bounding box. The eye rotation learner 130 may extract an image inside the adjusted bounding box as the eye area image. The eye rotation learner 130 may extract the iris area image after adjusting the size of the eye area image according to the method described above and calculate the phase cross correlation value.

The eye rotation learner 130 may train the third ANN model to generate the eye rotation value using the iris area image and the phase cross correlation value. The eye rotation value may refer to an angle of rotation clockwise or counterclockwise based on the central axis of the eye.

The eye coordinate learner 120 may train the second ANN model using the information generated from the first ANN model. In addition, the eye rotation learner 130 may train the third ANN model using the information generated from the second ANN model.

For example, the eye coordinate learner 120 may generate the training data using the multiple frame images input to the first ANN model and the information related to the head coordinates extracted from each frame image. The eye coordinate learner 120 may generate the eye area images from each frame image using the information related to the eye coordinates from the information related to the head coordinates. The eye coordinate learner 120 may train the second ANN model according to the process described above.

The eye rotation learner 130 may train the third ANN model further using the information related to the coordinates of the pupil center according to each frame image generated from the second ANN model. In addition, the eye rotation learner 130 may train the third ANN model by generating the training data according to the process described above using the image of the iris and/or pupil segmented by the eye coordinate learner 120.

The first to third ANN models may be trained independently from each other, and may also be trained using the information generated from each ANN model.

Hereinafter, the process of the balance function management system 10 according to the first exemplary embodiment generating the information on the balance function status and performing the balance function rehabilitation program using the trained ANN model will be described.

The balance function management system 10 may acquire frame images of n videos in real time from n cameras that capture a subject performing a balance function status check and/or a balance function rehabilitation program.

When capturing the subject with one camera, the camera may be set to a value greater than a preset frames per second (FPS). For example, one camera may capture the subject at 240 FPS.

When capturing the subject with a plurality of cameras, the balance function management system 10 may control the sync of the plurality of cameras through at least one processor. For example, the at least one processor may control the sync of the plurality of cameras in real time using a technology such as Genlock, etc.

The balance function management system 10 may acquire at least one of the head coordinates, the coordinates of the pupil center, and the information related to the eye phase changes of the subject using at least one of the first to third ANN models.

In addition, the balance function management system 10 may generate the information related to the head coordinates, the coordinates of the pupil center, and the eye phase changes using an algorithm in which at least one processor generates the training data of the first to third ANN models described above.

Hereinafter, it will be described that the information related to the head coordinates, the coordinates of the pupil center, and the eye phase changes is generated by using the first to third ANN models.

The head coordinate acquirer 140 executes the first ANN model stored in the memory 100, and inputs the frame image of the m-th video to the first ANN model to acquire the information related to the head coordinates according to the m-th video.

When capturing the subject with a plurality of cameras, the head coordinate acquirer 140 may independently input the frame images of the m-th video to the first ANN model.

For example, when capturing the subject with two cameras, the head coordinate acquirer 140 may input the frame image of the first video to the first ANN model. Thereafter, the head coordinate acquirer 140 may input the frame image of the second video to the first ANN model.

The head coordinate acquirer 140 may sequentially input the frame images of the m-th video to the first ANN model according to a predetermined order. For example, the head coordinate acquirer 140 may input a first frame image of the first video to the first ANN model and a first frame image of the second video to the first ANN model. Thereafter, the head coordinate acquirer 140 may input a second frame image of the first video to the first ANN model and a second frame image of the second video to the first ANN model. In this case, the frame image input to the first ANN model may include information on the extracted video.

The eye coordinate acquirer 150 may execute the second ANN model stored in the memory 100 and input the information related to the head coordinates to the second ANN model to acquire the information related to the coordinates of the pupil center according to the m-th video.

The phase change acquirer 160 executes the third ANN model stored in the memory 100 and inputs the information related to the coordinates of the pupil center according to the time sequence of the frame images of the m-th video to the third ANN model to acquire the information related to the eye phase changes according to the m-th video.

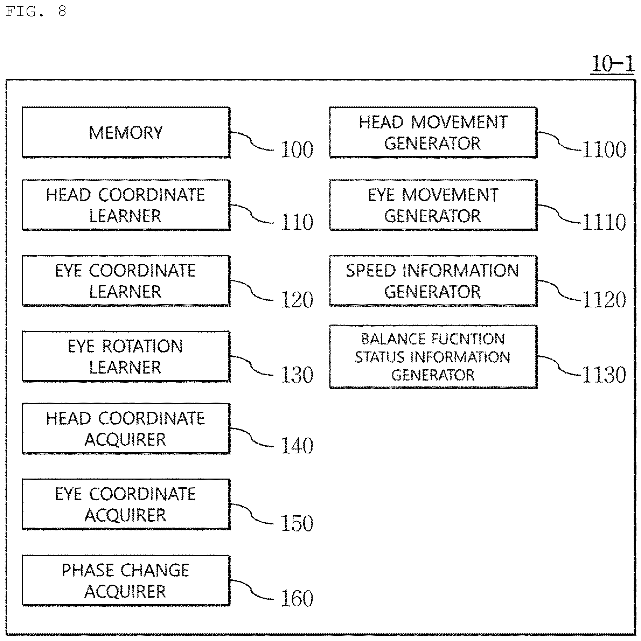

FIG. 8 is a block diagram of a balance function management system for generating information on a balance function status according to an exemplary embodiment of the present specification.

Referring to FIG. 8, a balance function management system 10-1 for generating balance function status information according to an exemplary embodiment of the present specification may include the memory 100, the head coordinate learner 110, the eye coordinate learner 120, the eye rotation learner 130, the head coordinate acquirer 140, the eye coordinate acquirer 150, the phase change acquirer 160, a head movement generator 1100, an eye movement generator 1110, a speed information generator 1120, and a balance function status information generator 1130. In describing the balance function management system 10-1, repetitive descriptions of each component will be omitted.

The head movement generator 1100 may generate the information related to the head movement in the m-th video using the information related to the head coordinates acquired from the first ANN model. The information related to the head movement may include horizontal movement and vertical movement of a head, and a degree of rotation of a head over time. The degree of rotation of the head may refer to a rotation angle of a head in the roll, pitch, and yaw directions.

The head movement generator 1100 may calculate a normal vector of a subject's head using the feature points of the head generated in each frame image and the coordinates of the feature points. The direction of the normal vector may refer to the direction in which the front of the subject's head faces. The head movement generator 1100 may calculate a normal vector of the head using the feature points of the head, based on the feature point of the tip of the nose among the feature points of the head.

Since calculating the normal vector for the front of the head using the feature point is a widely known technique among those skilled in the art, a detailed description thereof will be omitted.

The head movement generator 1100 may generate the information related to the head movement over time using the 3D head coordinate information and the normal vector according to the frame image of the m-th video.

The eye movement generator 1110 may generate the information related to the eye movement in the m-th video using the information related to the coordinates of the pupil center and the eye phase changes generated from the second ANN model and the third ANN model. The information related to the eye movement may include the information related to the movement of the pupil center in the vertical direction, the movement of the pupil center in the horizontal movement, and the eye rotation value over time.

The eye movement generator 1110 may calculate a gaze vector of an eye using the vertical coordinate value, horizontal coordinate value, and rotation value of the pupil center. Since calculating the gaze vector using the coordinate value and rotation value of the pupil center is a widely known technique among those skilled in the art, a detailed description thereof will be omitted.

The head movement generator 1100 and the eye movement generator 1110 may correct errors between the head movement and the eye movement information according to the training data and the head movement and the eye movement information of the subject.

The head movement and the eye movement information according to the training data may refer to actual data values for training the ANN model.

For example, the actual data values may refer to parameter values of the Euler angles of the head and eyes acquired by the eye coordinate learner 120. The eye coordinate learner 120 may set parameter values of the virtual camera to be similar to actual settings in the balance function status check and/or the balance function rehabilitation program. The eye coordinate learner 120 may change parameter values of the Euler angle to be similar to head and eye movements of the subject according to the balance function status check and/or the balance function rehabilitation program. In this case, the head and eye movement information of the virtual object according to the change in the parameter values of the head and eyes may refer to actual data values.

The head movement generator 1100 and the eye movement generator 1110 may correct the errors in the head movement and the eye movement between the frame images acquired within a preset time. For example, when the preset time is 1.5 seconds and the camera captures the subject at 100 FPS, the head movement generator 1100 and the eye movement generator 1110 may correct the errors in the head movement and the eye movement between 150 frame images.

The head movement generator 1100 and the eye movement generator 1110 may correct the errors of the head movement and the eye movement to have values within a preset range.

For example, the head movement generator 1100 and the eye movement generator 1110 may generate the information related to the head movement and the eye movement using the information related to the head coordinates, the coordinates of the pupil center, and/or the eye phase changes acquired from 150 frame images (frame images acquired for 1.5 seconds based on an arbitrary frame image) in the video in which the camera captures the subject at 100 FPS. In this case, the amount of head and eye movement at the time corresponding to the 50th frame image (frame image acquired 0.5 seconds after an arbitrary frame image) may be outside the preset error range. In this case, the head movement generator 1100 and the eye movement generator 1110 may calculate statistical values, such as the average or median of the amount of head and eye movement generated using information from the preceding frame image to replace the amount of movement at the time corresponding to the 50th frame image.

As another example, the head movement generator 1100 and the eye movement generator 1110 may correct the errors of the head and eye movements by applying a filter. For example, the head movement generator 1100 and the eye movement generator 1110 may correct the errors using filters such as a chaining Kalman filter, a moving average filter, a Savitzky-Golay filter, a high pass filter, a low pass filter, and a band pass filter, etc.

The speed information generator 1120 may generate the information related to the head and eye movement speeds in the m-th video using the information related to the head movement and the eye movement. The speed information generator 1120 may calculate the vertical movement speed of the head, the horizontal movement speed of the head, and/or the rotation speed of the head over time using the information related to the head movement. The speed information generator 1120 may calculate the vertical movement speed of the eye, the horizontal movement speed of the eye, and/or the rotation speed of the eye over time using the information related to the eye movement.

The speed information generator 1120 may filter out noise values from the information related to the head and eye movement speeds. The speed information generator 1120 may remove noise from the information related to the head and eye movement speeds using the filters such as a chaining Kalman filter, a moving average filter, a Savitzky-Golay filter, a high pass filter, a low pass filter, or a band pass filter, etc.

According to an exemplary embodiment of the present specification, the speed information generator 1120 may generate the information related to the head movement speed and the eye movement speed within a preset time based on a time when the head movement becomes greater than or equal to a preset threshold value. In the balance function status check, the subject may move the head in a horizontal (lateral left, lateral right) direction. In this case, the speed information generator 1120 may generate the information related to the head and eye movement speeds when the movement of the head in the horizontal direction is greater than or equal to the preset threshold value.

In addition, the subject may move the head in the lower right and upper right directions while the right side of the face is turned toward the front of the subject so that a right anterior semicircular canal and a left posterior semicircular canal (right anterior, left posterior (RALP)) are stimulated. In addition, the subject may move the head in the left-down and left-up directions while the left side of the face is turned toward the front of the subject so that the right posterior semicircular canal and the left anterior semicircular canal (left anterior, right posterior (LARP)) are stimulated. In this case, the speed information generator 1120 may generate the information related to the head and eye movement speeds when the movement of the head in the vertical direction is greater than or equal to the preset threshold value.

Hereinafter, the direction in which the subject rotates the head so that the RALP is stimulated will be described as the RALP direction, and the direction in which the subject rotates the head so that the LARP is stimulated will be described as the LARP direction.

For example, the speed information generator 1120 may determine whether the head movement is greater than or equal to a threshold value by using the head movement information according to a frame image existing within a preset time based on the last input frame image. The speed information generator 1120 may calculate the difference between the maximum and minimum values of the coordinates of the feature points of the head in the head movement information according to the frame image existing within the preset time to determine whether the head movement is greater than or equal to the threshold value.

As another example, the memory 100 may further store a fourth ANN model that generates the information on the head movement. The fourth ANN model may be trained by allowing at least one processor to use the frame image of the video performing the balance function status check and the data of the pitch and yaw values of the head in the corresponding frame image. The fourth ANN model may be a time series model or a transformer model. In this case, the frame image may be labeled with information on the lateral, RALP, and LARP directions. The speed information generator 1120 may input a frame image existing within a preset time based on the last acquired frame image to the fourth ANN model to confirm whether the head movement is greater than or equal to the threshold value.

The speed information generator 1120 may calculate the head and eye movement speeds using the information related to the head and eye movement generated during a preset time after the time when the head movement becomes greater than or equal to the threshold value. For example, the speed information generator 1120 may calculate the head and eye movement speeds using the information related to the head and eye movement generated within 1.5 seconds after the time when the head movement becomes greater than or equal to the threshold value.

The speed information generator 1120 may display the information related to the head and eye movement speeds on the display device.

The balance function status information generator 1130 may generate the information on the balance function status of the subject using the information related to the head and eye movement speeds.

According to an exemplary embodiment of the present specification, the balance function status information generator 1130 may calculate a gain coefficient using a time value (head peak index) when the head movement speed of the subject is relatively the largest within a preset analysis window and a time value (eye peak index) when the eye movement speed is relatively the largest when the eye of the subject moves in the direction of the head movement and then returns to the original position. The analysis window may mean a preset time range based on the time when the head movement becomes greater than or equal to the threshold value.

The balance function status information generator 1130 may calculate the gain coefficient using [Equation 1] using the head peak index and the eye peak index from the information related to the head and eye movement speeds from which noise has been removed.

[Equation 1]

$$\text{Gain Coefficient} = \frac{Window\ Size - |Phase\ Shift|}{Window\ Size}$$

- Window Size: Size (time) of analysis window
- Phase Shift: Difference between Head Peak Index and Eye Peak Index

Thereafter, the balance function status information generator 1130 may calculate gain using the gain coefficient.

[Equation 2]

$$\text{Gain} = \frac{Eye\ Extrema}{Head\ Extrema} \times Gain\ coefficient$$

- Eye Extrema: Maximum Speed of Pupil
- Head Extrema: Maximum Speed of Head

The balance function status information generator 1130 may calculate the gains for the left eye and the right eye, respectively.

In addition, the balance function status information generator 1130 may calculate the gains in the m-th video, respectively. For example, when the subject is captured using two cameras, the gains for the two videos may be calculated, respectively. In this case, the gains of the left eye and the right eye in the first video and the gains of the left eye and the right eye in the second video may be calculated. The balance function status information generator 1130 may calculate a statistical value for the gain of the left eye calculated in the first video and the second video, and calculate at least one statistical value for the gain of the right eye calculated in the first video and the second video. The statistical value may correspond to an average, a median, a minimum, a maximum, a standard deviation, etc.

According to an exemplary embodiment of the present specification, when the subject is captured by a plurality of cameras (n is 2 or more), the head movement generator 1100 may further generate reference head movement information by calculating the statistical values of the information related to the head coordinates according to the m-th video. The eye movement generator 1110 may further generate the reference eye movement information by calculating the statistical values of the information related to the coordinates of a pupil center and the eye phase changes according to the m-th video.

When the subject is captured by the plurality of cameras, a difference in the coordinate values of the 3D head generated from the frame images of the m-th video may occur depending on the position, angle, etc., of the cameras.

The head movement generator 1100 may generate the reference head coordinate information by calculating the average value of the coordinates of each feature point in the three-dimensional head coordinates generated from the frame images that are synchronized with each other in the m-th video. The head movement generator 1100 may further generate the information related to the reference head movement using the reference head coordinate information over time.

The eye movement generator 1110 may generate the information related to the reference coordinates of the pupil center and the reference eye phase change by calculating an average value of the information related to the coordinates of the pupil center and the eye phase change generated from the frame images that are synchronized in the m-th video. The eye movement generator 1110 may generate a reference gaze vector of the eye using the information related to the reference coordinates of the pupil center and the reference eye phase change. The eye movement generator 1110 may further generate the information related to the reference eye movement using the reference coordinates of the pupil center, the information related to the reference eye phase change, and the reference gaze vector.

In this case, the speed information generator 1120 may generate the information related to the head movement speed and the eye movement speed for the m-th video, respectively, within a preset time based on the time when the reference head movement becomes greater than or equal to the preset threshold value.

The balance function status information generator 1130 may calculate the gains using the head peak index and the eye peak index for the m-th video within the preset time based on the time when the reference head movement becomes greater than or equal to the preset threshold value.

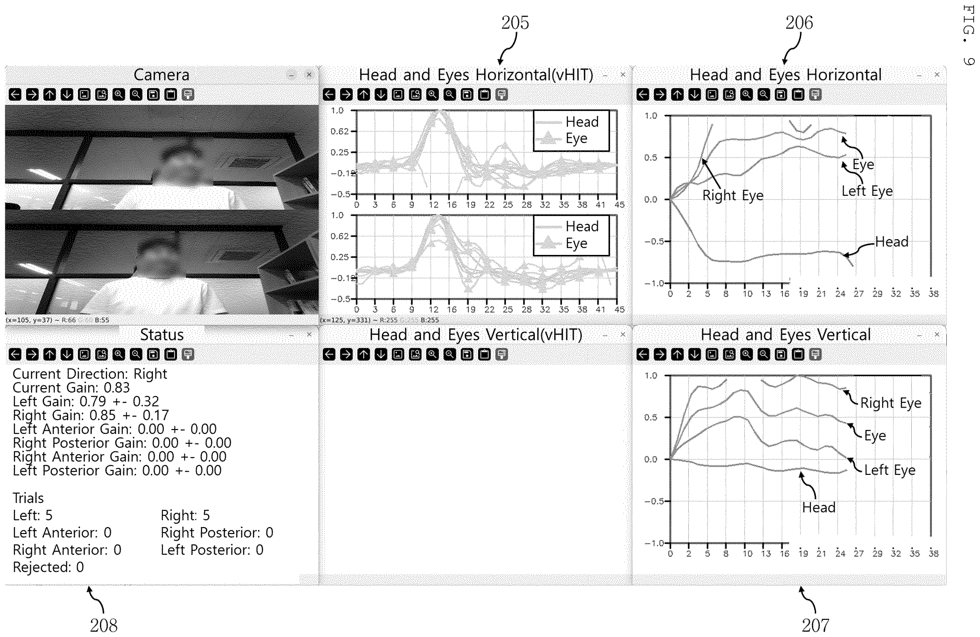

FIG. 9 is a diagram illustrating an example of outputting information on a balance function status to a display according to an exemplary embodiment of the present specification.

Referring to FIG. 9, the head movement generator 1100, the eye movement generator 1110, the speed information generator 1120, and the balance function status information generator 1130 may output the calculated information on the display screen. On the display screen, videos captured by the plurality of cameras may be outputted, respectively.

The speed information generator 1120 may output the information on the head and eye movement speeds for the m-th video as a graph 205, respectively. In the graph 205 of the head and eye movement speeds, the speed information according to the number of times of balance function status checks may be superimposed and displayed. In the speed graph 205 of the head and eye movements, the time values at which a peak and/or valley appear may correspond to the head peak index and/or the eye peak index. In the graph, the vertical axis may correspond to the speed value, and the horizontal axis may correspond to the time value.

The head movement generator 1100 and the eye movement generator 1110 may output the head and eye movement information as graphs. The head movement generator 1100 and the eye movement generator 1110 may output a movement graph 206 of the head and eye in a horizontal direction and a movement graph 207 of the head and eye in a vertical direction. In addition, the movement graph for the rotation direction of the head and/or eye may be further output. In addition, the head and eye movement graphs may include the movement information for the m-th video, and may include the reference head movement and reference eye movement information.

The balance function status information generator 1130 may output information 208 related to the balance function status check for the subject to a display device. The information related to the balance function status check may include at least one of the rotation direction (lateral, RALP, LARP) of the head, a gain and standard deviation according to the head rotation direction, the number of balance function status checks according to the rotation direction of the head, the number of times of successful calculations of the gain, and the number of times of failures in the calculation of the gain.

In addition, the balance function status information generator 1130 may generate information on whether the semicircular canal is abnormal according to the gain. For example, the balance function status information generator 1130 may generate information on the abnormality of the semicircular canal when the gains of the left and right eyes are lower than or equal to the preset value in the balance function status check.

In addition, the subject may rotate his/her head in the lateral direction in the balance function status check. In this case, when the difference in the gains of the left and right eyes when the subject turns his/her head to the left and to the right is greater than or equal to the preset value, the balance function status information generator 1130 may generate abnormal information of the semicircular canal.

In addition, in the balance function status check, the subject may rotate his/her head in the RALP or LARP direction. In this case, when the difference in the gains of the left and right eyes when the subject turns his/her head upward and downward is greater than or equal to the preset value, the balance function status information generator 1130 may generate abnormal information of the RALP or LARP.

In addition, the balance function status information generator 1130 may further generate the information on whether there is an abnormality in the central vestibular nerve function and an abnormality in the peripheral vestibular nerve function by using the information related to the eye movement and the gain information.

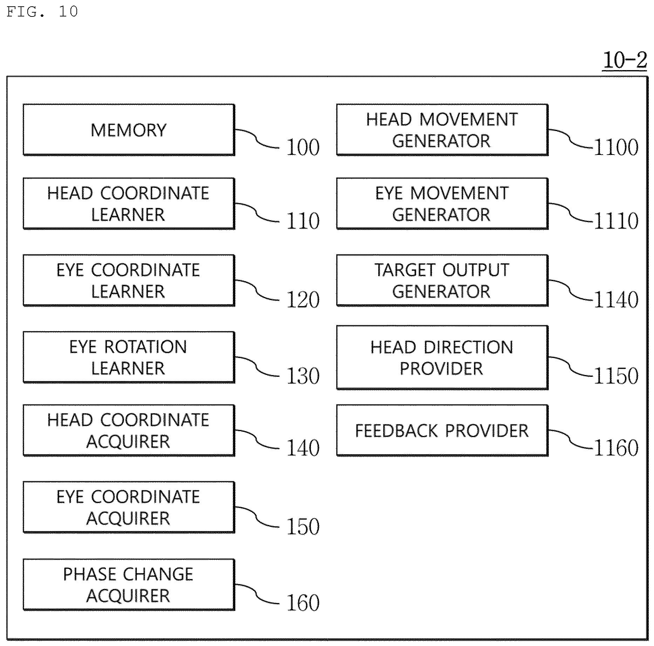

FIG. 10 is a block diagram of a balance function management system for performing a balance function rehabilitation program according to an exemplary embodiment of the present specification.

Referring to FIG. 10, a balance function management system 10-2 for performing a balance function rehabilitation program according to an exemplary embodiment of the present specification may include the memory 100, the head coordinate learner 110, the eye coordinate learner 120, the eye rotation learner 130, the head coordinate acquirer 140, the eye coordinate acquirer 150, the phase change acquirer 160, the head movement generator 1100, the eye movement generator 1110, a target output generator 1140, a head direction provider 1150, and a feedback provider 1160. In describing the balance function management system 10-2, repetitive descriptions of each component will be omitted.

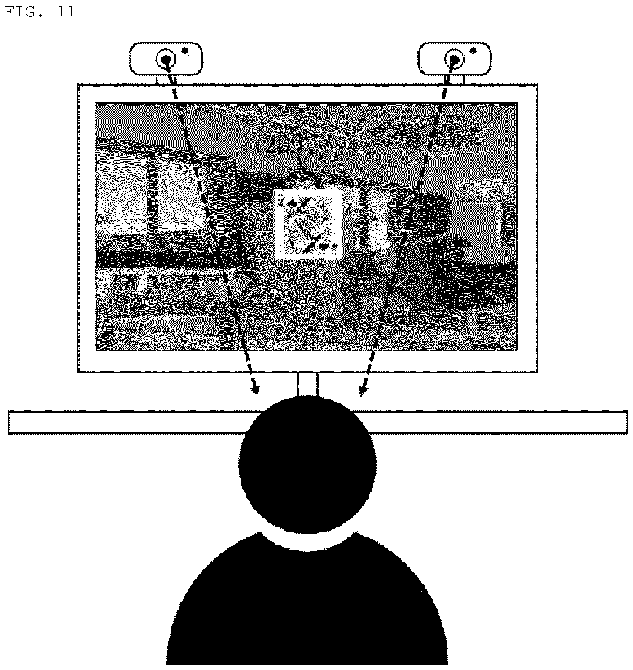

FIG. 11 is a diagram illustrating an example of a scene performing the balance function rehabilitation program according to an exemplary embodiment of the present specification.

Referring to FIG. 11, the target output generator 1140 may output a virtual target 209 to the display device. The virtual target may be displayed at any position on the display device.

The head direction provider 1150 may provide the subject with the information on the rotation direction of the head according to the balance rehabilitation protocol.

In addition, the head direction provider 1150 may provide information so that the subject returns to a state before rotating the head within a preset time after rotating the head.

The head direction provider 1150 may visually display the information on the display device. In addition, the head direction provider 1150 may output the information to an audio device.

The feedback provider 1160 may provide feedback according to the head movement and the eye movement of the subject.

According to the balance rehabilitation protocol, the reference of the angle at which the subject should rotate the head may be determined in advance. The feedback provider 1160 may compare the rotation angle of the subject's head generated from the head movement generator 1100 with the reference of the angle. The feedback provider 1160 may provide different feedbacks depending on whether the rotation angle of the subject's head satisfies the reference of the angle.

In addition, the eye movement generator 1110 may generate the coordinate information of the gaze point which the subject's gaze faces on the display device using the eye gaze vector. The feedback provider 1160 may compare the coordinate information of the gaze point generated from the eye movement generator 1110 with the coordinate information of the virtual target 209.

When the coordinates of the gaze point are positioned within the area of the virtual target 209, the feedback provider 1160 may change the color value of the virtual target 209. In addition, the feedback provider 1160 may further display the gaze point within the virtual target 209.

When the coordinates of the gaze point are positioned outside the area of the virtual target 209, the feedback provider 1160 may display the position of the gaze point on the display device.

When the subject is captured by the plurality of cameras, the feedback provider 1160 may provide the feedback according to the information related to the reference head movement and the reference eye movement.

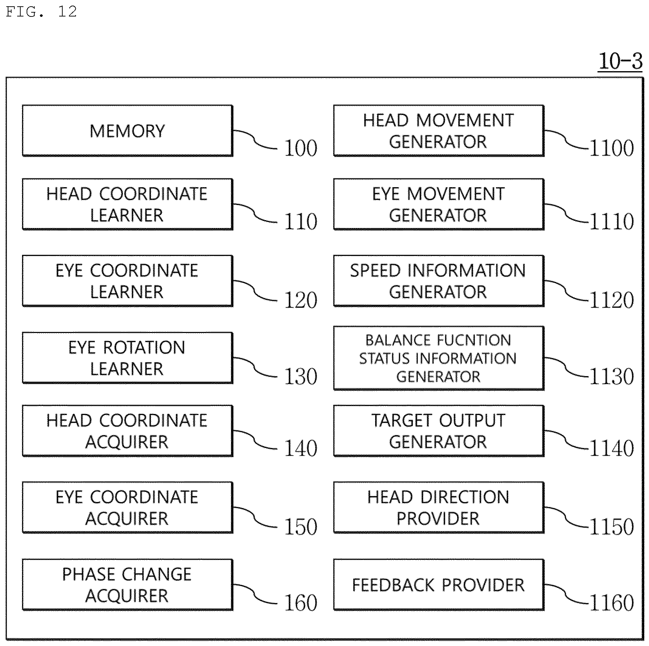

FIG. 12 is a block diagram of a balance function management system for generating information on a balance function status and performing a balance function rehabilitation program according to an exemplary embodiment of the present specification.

Referring to FIG. 12, the balance function management system 10-3 may include the memory 100, the head coordinate learner 110, the eye coordinate learner 120, the eye rotation learner 130, the head coordinate acquirer 140, the eye coordinate acquirer 150, the phase change acquirer 160, the head movement generator 1100, the eye movement generator 1110, the speed information generator 1120, the balance function status information generator 1130, the target output generator 1140, the head direction provider 1150, and the feedback provider 1160. The balance function management system 10-3 may generate a gain to provide a balance function rehabilitation program according to whether the balance function is abnormal.

Hereinafter, a balance function management system according to a second exemplary embodiment of the present specification will be described. Hereinafter, n may mean a natural number greater than or equal to 2.

In describing the second exemplary embodiment, the repeated description in the first embodiment will be omitted.

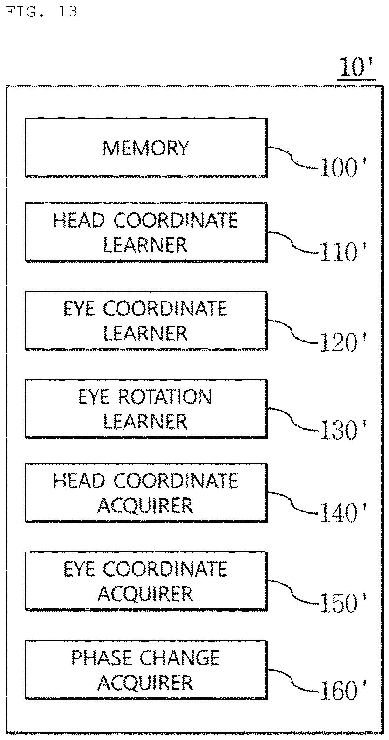

FIG. 13 is a block diagram of a balance function management system according to a second exemplary embodiment of the present specification.

Referring to FIG. 13, a balance function management system 10' according to the second exemplary embodiment of the present specification may include a memory 100', a head coordinate learner 110', an eye coordinate learner 120', an eye rotation learner 130', a head coordinate acquirer 140', an eye coordinate acquirer 150', and a phase change acquirer 160'.

The memory 100' may store at least one of a first ANN model that generates information related to head coordinates, a second ANN model that acquires information related to coordinates of a pupil center, and a third ANN model that acquires information related to eye phase changes.

According to an exemplary embodiment of the present specification, the first ANN model may be trained by allowing the head coordinate learner 110' to use facial feature points and coordinates of the feature points extracted from frame images of multiple videos in which a human face is captured as training data.

The head coordinate learner 110' may extract feature points after adjusting the sync of the multiple videos when extracting the feature points from the multiple videos in which a person is captured using a plurality of cameras.

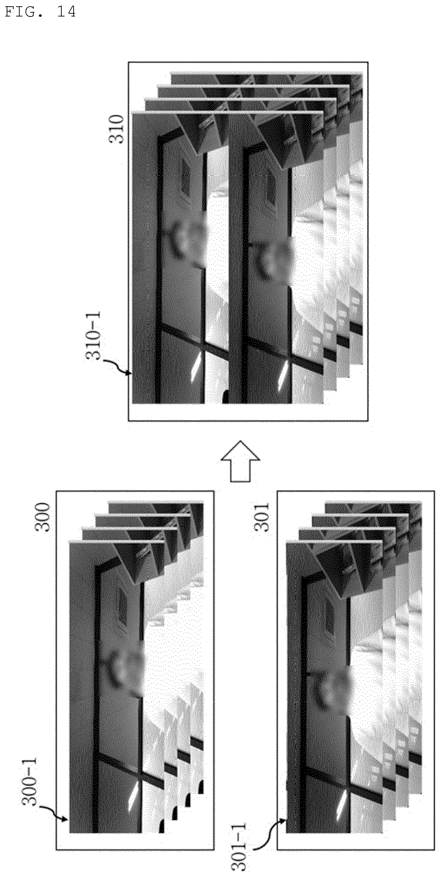

FIG. 14 is a reference diagram illustrating an example in which a head coordinate learner concatenates frame images according to an exemplary embodiment of the present specification.

Referring to FIG. 14, the video in which the human face is captured may be a video captured by two cameras. The head coordinate learner 110' may adjust the sync of the first video 300 captured by the first camera and the second video 301 captured by the second camera. The head coordinate learner 110' may generate a multi-frame image 310 by concatenating frame images having the same sync in the first video 300 and the second video 301. For example, the first frame image 300-1 of the first video 300 and the first frame image 301-1 of the second video 301 may be concatenated to generate a first multi-frame image 310-1. The multi-frame image may refer to one image generated by concatenating multiple frame images having the same sync.

Hereinafter, the multi-frame image will be described assuming that the frame images of the m-th video having the same sync among the frame images of n videos are concatenated.

The head coordinate learner 110' may generate a multi-frame image by concatenating the frame images of the m-th video having the same sync among n videos captured by n cameras. In addition, the head coordinate learner 110' may label information of the video from which the frame images are extracted.

The head coordinate learner 110' may extract feature points for a human face from the multi-frame image. For example, the head coordinate learner 110' may extract feature points positioned on a human face from each multi-frame image.

The head coordinate learner 110' may train the first ANN model to generate 3D head coordinates for each multi-frame image using training data including feature points extracted from each multi-frame image and coordinates of the feature points.

The head coordinate learner 110' may train the first ANN model to generate the 3D coordinates of the head for the frame image of the m-th video in the multi-frame image, respectively.

In addition, the head coordinate learner 110' may train the first ANN model to generate the reference head coordinate for the three-dimensional coordinates of the head. The reference head coordinate may refer to an average value of the coordinates of each feature point in the three-dimensional coordinates of the head generated from the frame image of the m-th video.

In addition, the head coordinate learner 110' may train the first ANN model to further generate the head movement information using the 3D coordinates extracted from each multi-frame image.

In addition, the head coordinate learner 110' may train the first ANN model further using, as training data, data on a 3D standard head model, such as the frame image from which the feature points are extracted, a 3DMM, a FLAME model.

According to an exemplary embodiment of the present specification, the head coordinate learner 110' may use feature points having coordinate values according to preset criteria as training data.

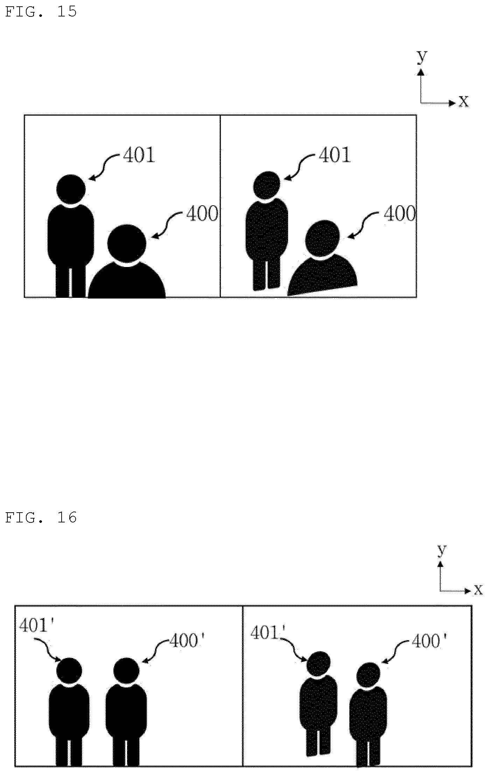

FIG. 15 is a diagram illustrating an example of a multi-frame image of a scene where a subject performing a balance function status check and/or a balance function rehabilitation program according to an exemplary embodiment of the present specification is captured.

Referring to FIG. 15, the multiple videos in which the person is captured may be multiple videos of performing the balance function status check and/or the balance function rehabilitation program using the balance function management system 10'.

The head coordinate learner 110' may extract the facial feature points and the coordinates of the feature points of the subject 400 from the multi-frame image to train the first ANN model.

As described above, since the subject 400 is sitting on a chair and the examiner 401 is standing, the feature points extracted from the face of the subject 400 may be positioned relatively lower than the feature points extracted from the face of the examiner 401. Since the process of extracting the feature points from the face of the subject has been described above, a detailed description thereof will be omitted.

FIG. 16 is a diagram illustrating an example of the multi-frame image of the scene where the subject performing the balance function status check and/or the balance function rehabilitation program according to an exemplary embodiment of the present specification is captured.

Referring to FIG. 16, in the video, the balance function status check and/or the balance function rehabilitation program may be performed while both the subject 400' and examiner 401' are standing. In this case, the head coordinate learner 110' may extract the feature points having the coordinate values that are relatively closer to the center of the display screen as training data.

The head coordinate learner 110' may generate the feature points and the coordinate information of the feature points from the face of the subject included in the frame image of the m-th video in the multi-frame image, respectively.

The head coordinate learner 110' may track the coordinates of the feature points extracted from the frame image preceding an arbitrary frame image of the m-th video. When the feature points are not extracted from the face of the subject in the frame image of the m-th video, the head coordinate learner 110' may extract the training data using the image of the subject in the preceding frame image.

The eye coordinate learner 120' may generate the multi-frame image by controlling the sync of the multiple videos. Alternatively, the eye coordinate learner 120' may receive the multi-frame image generated from the head coordinate learner 110'.

The eye coordinate learner 120' may extract an eye area image of a subject, which is an image of an area including an eye of the subject, from the multi-frame image. The eye coordinate learner 120' may train the second ANN model to generate the information related to the coordinates of the pupil center by using the eye area image of the subject extracted from each frame image as training data.

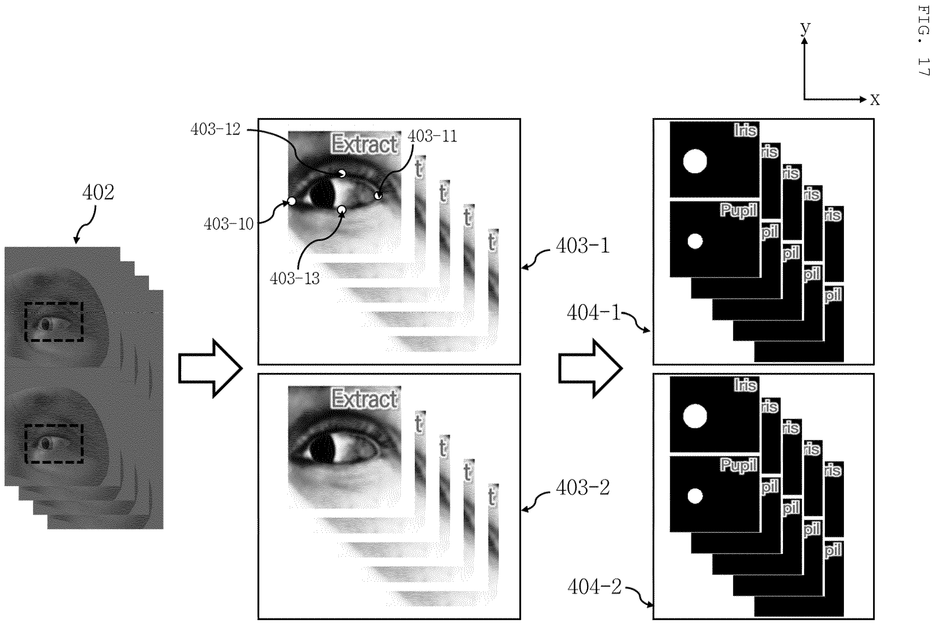

FIG. 17 is a diagram illustrating an example of preprocessing an eye area image according to an exemplary embodiment of the present specification.

Referring to FIG. 17, the eye coordinate learner 120' may extract eye area images 403-1 and 403-2 according to the frame images of the m-th video from each multi-frame image 402. The eye coordinate learner 120' may segment the iris and pupil areas from the eye area images 403-1 and 403-2.

The eye coordinate learner 120' may estimate an area for a part where the iris and/or pupil are covered by an eyelid.

The eye coordinate learner 120' may train the second ANN model using data in which the iris and/or pupil areas are segmented in the eye area images 403-1 and 403-2. For example, the eye coordinate learner 120' may generate mask image 404-1 and 404-2 in which the area occupied by the iris and/or pupil and the remaining area have different pixel values in the eye area images 403-1 and 403-2.

The eye coordinate learner 120' may train the second ANN model to generate the information related to the coordinates of the pupil center for frame images of each video using the mask images 404-1 and 404-2 and/or two-dimensional pixel values of the mask images 404-1 and 404-2 as training data.

As another example, the eye coordinate learner 120' may train the second ANN model using a heatmap model that segments and displays the iris and/or pupil area in the eye area images 403-1 and 403-2.

According to an exemplary embodiment of the present specification, the eye coordinate learner 120' may train the second ANN model to generate the eye feature points and coordinate information of the feature points from the eye area images 403-1 and 403-2, and to generate the horizontal coordinate values and vertical coordinate values of the pupil center for the frame images of each video using the coordinates of the plurality of preset feature points. The eye coordinate learner 120' may extract normalized coordinates of a pupil center using the coordinates of the plurality of feature points extracted from the eye area images 403-1 and 403-2.

For example, the eye coordinate learner 120' may extract horizontal coordinates of a normalized pupil center using a feature point 403-10 for a medial canthus and a feature point 403-11 for an outer canthus among the feature points extracted from the eye area image 403-1.

In addition, the eye coordinate learner 120' may extract vertical coordinates of the normalized pupil center using a feature points 403-12 for an upper eyelid and a feature point 403-13 for a lower eyelid among the feature points extracted from the eye area image 403-1.

In FIG. 17, the feature point 403-10 for the medial canthus and the feature point 403-11 for the outer canthus are illustrated as being positioned on the same horizontal line, and the upper eyelid feature point 403-12 and the lower eyelid feature point 403-13 are illustrated as being positioned on the same vertical line. However, this may vary depending on a capturing angle of a camera, a head angle of a persona, etc. In this case, the eye coordinate learner 120' may transform the image so that in the rotating eye area image, the feature point 403-10 for the medial canthus and the feature point 403-11 for the outer canthus are positioned on the same horizontal line, and the upper eyelid feature point 403-12 and the lower eyelid feature point 403-13 are positioned on the same vertical line. In this case, the eye coordinate learner 120' may transform the rotating eye area image using an affine transform, etc.

The eye coordinate learner 120' may extract the coordinates of the feature points and the eye feature points from each eye area image 403-1 or 403-2, and generate the coordinate information of the normalized pupil centers for the frame images of each video. The eye coordinate learner 120' may train the second ANN model further using the horizontal coordinate values and the vertical coordinate values of the normalized pupil center for the generated frame images of each video as the training data.

In addition, the eye coordinate learner 120' may calculate the average value of the horizontal coordinate value and the vertical coordinate value of the pupil center calculated from the multi-frame image. The eye coordinate learner 120' may train the second ANN model further using the average value of the horizontal coordinate values and the vertical coordinate values of the pupil center as training data.

According to another exemplary embodiment of the present specification, the memory 100' may store data of at least one virtual object. The second ANN model may be trained to generate information related to the coordinates of the pupil center by allowing the eye coordinate learner 120' to use training data that includes a parameter value obtained by changing at least one of parameters related to head rotation, eye rotation, and camera settings of a parameter of a virtual object and an image of the virtual object acquired according to the parameter value. Since the process of training the second ANN model using virtual object has been described above, a detailed description thereof will be omitted.

In addition, the memory 100' may store labeling data including at least one of head coordinates, coordinates of a pupil center, and information related to camera settings according to an image in which a human face is captured. The eye coordinate learner 120' may train the second ANN model using the data.

In addition, the eye coordinate learner 120' may train the second ANN model using at least one of training data using the eye area image, training data obtained according to parameter changes of the virtual object, and labeling data.

According to an exemplary embodiment of the present specification, the eye rotation learner 130' may extract an eye area image. The eye rotation learner 130' may train the third ANN model to generate an eye rotation value using training data including information related to the eye phase changes generated according to the time sequence of the eye area image according to each video.

The eye rotation learner 130' may generate the multi-frame image by controlling the sync of the multiple videos. Alternatively, the eye rotation learner 130' may receive the multi-frame image generated from the head coordinate learner 110'.

The eye rotation learner 130' may extract an eye area image for a frame image of the m-th video from the multi-frame image. The eye rotation learner 130' may generate the information related to the eye phase changes by comparing the eye area image extracted from the multi-frame image with eye area images extracted from multi-frame images within a preset time range based on the multi-frame image. In this case, the eye rotation learner 130' may generate the information related to the eye phase changes according to the m-th video using the eye area image for the m-th video.

According to an exemplary embodiment of the present specification, the eye rotation learner 130' may extract an iris area image. The eye rotation learner 130' may train the third ANN model using information related to a phase change of the iris according to the time sequence of the iris area image.

More specifically, the eye rotation learner 130' may compare an iris area image extracted from an arbitrary multi-frame image with iris area images extracted from multi-frame images within a preset time range based on an arbitrary multi-frame image. The iris area image may be a mask image in which an area occupied by an iris is distinguished by different pixel values from other areas.

In addition, the eye rotation learner 130' may generate the information related to the phase change using the mask image of the iris generated by the eye coordinate learner 120'.

The eye rotation learner 130' may generate the information related to the phase change of the iris by comparing the pixel values of the iris area image extracted from the arbitrary multi-frame image with those of other iris area images. The eye rotation learner 130' may generate phase cross correlation values for the pixel values of the iris area image extracted from the arbitrary multi-frame image and other iris area images by using phase cross correlation analysis.

The eye rotation learner 130' may generate the phase cross correlation value by a method of obtaining a cross correlation value upsampled by a fast Fourier transform (FFT).

The eye rotation learner 130' may train the third ANN model using the image and the phase cross correlation value of the iris area as the training data.

According to an exemplary embodiment of the present specification, the eye rotation learner 130' may calculate the size of the area occupied by the pupil in the eye area image.

The eye rotation learner 130' may calculate the phase cross correlation value after adjusting the sizes of each eye area image. Since the process of calculating the phase cross correlation value after adjusting the sizes of each eye area image has been described above, a detailed description thereof will be omitted.

In addition, the eye rotation learner 130' may generate a bounding box of an area including an eye in the multi-frame image. The eye rotation learner 130' may extract the pupil center within the bounding box. Alternatively, the eye rotation learner 130' may receive the information related to the coordinates of the pupil center generated from the second ANN model.

The eye rotation learner 130' may train the third ANN model to generate the eye rotation value for the m-th video using the iris area image and the phase cross correlation value. In addition, the eye rotation learner 130' may calculate an average value of eye rotation values calculated from the frame images in the m-th video over time. The eye rotation learner 130' may train the third ANN model to generate the reference eye rotation value using the average value.

The eye rotation learner 130' may train the third ANN model to generate the information related to the phase changes of the left and right eyes by extracting the eye area images for the left and right eyes from the frame image.

The eye coordinate learner 120' may train the second ANN model using the information generated from the first ANN model. In addition, the eye rotation learner 130' may train the third ANN model using the information generated from the second ANN model.

The first to third ANN models may be trained independently from each other, and may also be trained using the information generated from each ANN model.

Hereinafter, the process in which the balance function management system 10' generates the balance function status information and performs the balance function rehabilitation program using the trained ANN model will be described.

The balance function management system 10' may acquire frame images of n videos in real time from n cameras that capture a subject performing a balance function status check and/or a balance function rehabilitation program.

The balance function management system 10' may control the sync of the plurality of cameras through at least one processor.

The head coordinate acquirer 140' may execute the first ANN model stored in the memory 100' and input the multi-frame image concatenating the frame images of n videos to the first ANN model to acquire the information related to the head coordinates according to the m-th video.

The eye coordinate acquirer 150' may execute the second ANN model stored in the memory 100' and input the information related to the head coordinates to the second ANN model to acquire the information related to the coordinates of the pupil center according to the m-th video.

The phase change acquirer 160' may execute the third ANN model stored in the memory 100' and input the information related to the coordinates of the pupil center of the m-th video according to the time sequence of the multi-frame images to the third ANN model to acquire the information related to the eye phase changes according to the m-th video.

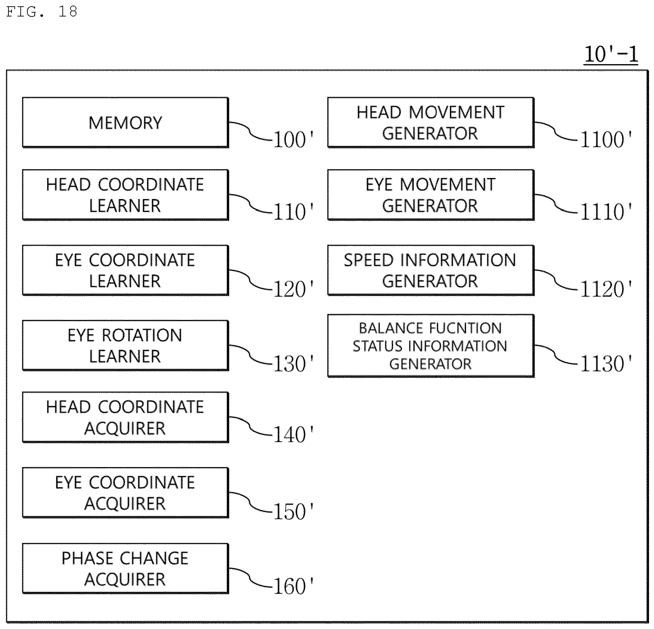

FIG. 18 is a block diagram of a balance function management system for generating information on a balance function status according to an exemplary embodiment of the present specification.

Referring to FIG. 18, a balance function management system 10'-1 for generating balance function status information according to an exemplary embodiment of the present specification may include the memory 100', the head coordinate learner 110', the eye coordinate learner 120', the eye rotation learner 130', the head coordinate acquirer 140', the eye coordinate acquirer 150', the phase change acquirer 160', a head movement generator 1100', an eye movement generator 1110', a speed information generator 1120', and a balance function status information generator 1130'. In describing the balance function management system 10'-1, repetitive descriptions of each component will be omitted.

The head movement generator 1100' may generate the information related to the head movement in the m-th video using the information related to the head coordinates acquired from the first ANN model.

The head movement generator 1100' may calculate a normal vector of a subject's head using the feature points of the head generated in each frame image and the coordinates of the feature points. The head movement generator 1100' may calculate a normal vector of the head using the feature points of the head, based on the feature point of the tip of the nose among the feature points of the head.

The head movement generator 1100' may generate the information related to the head movement over time using the 3D head coordinate information and the normal vector according to the frame image of the m-th video.

The eye movement generator 1110' may generate the information related to the eye movement in the m-th video using the information related to the coordinates of the pupil center and the eye phase changes generated from the second ANN model and the third ANN model.

The eye movement generator 1110' may calculate a gaze vector of an eye using the vertical coordinate value, horizontal coordinate value, and rotation value of the pupil center.

The head movement generator 1100' and the eye movement generator 1110' may correct errors between the head movement and the eye movement information according to the training data and the head movement and the eye movement information of the subject.

Since the process of correcting errors has been described above, a detailed description thereof will be omitted.

Alternatively, the head coordinate learner 110', the eye coordinate learner 120', and the eye rotation learner 130' may train the first to third ANN models to correct the error.

The speed information generator 1120' may generate the information related to the head and eye movement speeds in the m-th video using the information related to the head movement and the eye movement.

The speed information generator 1120' may filter out noise values from the information related to the head and eye movement speeds. Since the process of filtering out noise values from the information related to the head and eye movement speeds has been described above, a detailed description thereof will be omitted.

According to an exemplary embodiment of the present specification, the speed information generator 1120' may generate the information related to the head movement speed and the eye movement speed within a preset time based on a time when the head movement becomes greater than or equal to a preset threshold value. Since the process of generating the information related to the head movement speed and the eye movement speed has been described above, a detailed description thereof will be omitted.

The speed information generator 1120' may display the information related to the head and eye movement speeds on the display device.

The balance function status information generator 1130' may generate the information on the balance function status of the subject using the information related to the head and eye movement speeds.

According to an exemplary embodiment of the present specification, the balance function status information generator 1130' may calculate a gain coefficient using a head peak index and an eye peak index.

The balance function status information generator 1130' may calculate the gain coefficient using the above [Equation 1] using the head peak index and the eye peak index from the information related to the head and eye movement speeds from which noise has been removed.

Thereafter, the balance function status information generator 1130' may calculate gain using the above [Equation 2].

The balance function status information generator 1130' may calculate the gains for the left eye and the right eye, respectively.

The balance function status information generator 1130' may calculate the gains in the m-th video, respectively.

According to an exemplary embodiment of the present specification, the head movement generator 1100' may further generate the reference head movement information by calculating the statistical values of the information related to the head coordinates according to the m-th video generated from the multi-frame image.

The eye movement generator 1110' may further generate the reference eye movement information by calculating the statistical values of the information related to the coordinates of a pupil center and the eye phase changes according to each video generated from the multi-frame image.

The head movement generator 1100' may generate the reference head coordinate information by calculating the average value of the three-dimensional head coordinates generated from the frame images that are synchronized with each other in the m-th video. The head movement generator 1100' may further generate the information related to the reference head movement using the reference head coordinate information over time.

Alternatively, the head coordinate acquirer 110' may acquire the reference head coordinates from the first ANN model. The head movement generator 1100' may further generate the information related to the reference head movement using the reference head coordinates.

The eye movement generator 1110' may generate the information related to the reference coordinates of the pupil center and the reference eye phase change by calculating an average value of the information related to the coordinates of the pupil center and the eye phase change generated from the frame images that are synchronized in the m-th video. The eye movement generator 1110' may generate a reference gaze vector of the eye using the information related to the reference coordinates of the pupil center and the reference eye phase change. The eye movement generator 1110' may further generate the information related to the reference eye movement using the reference coordinates of the pupil center, the information related to the reference eye phase change, and the reference gaze vector.

Alternatively, the eye coordinate acquirer 150' and the phase change acquirer 160' may acquire the information related to the reference coordinates of a pupil center and the rotation value information of the reference eye from the second and third ANN models. The eye movement generator 1110' may generate the information related to the reference eye movement using the information related to the reference coordinates of a pupil center and the rotation value information of the reference eye.

In this case, the speed information generator 1120' may generate the information related to the head movement speed and the eye movement speed for the m-th video, respectively, within a preset time based on the time when the reference head movement becomes greater than or equal to the preset threshold value.

The balance function status information generator 1130' may calculate the gains, respectively, using the head peak index and the eye peak index for the m-th video within the preset time based on the time when the reference head movement becomes greater than or equal to the preset threshold value.

As illustrated in FIG. 9, the head movement generator 1100', the eye movement generator 1110', the speed information generator 1120', and the balance function status information generator 1130' may output the calculated information on the display screen.

Since the information related to the balance function status check has been described above, a detailed description thereof will be omitted.

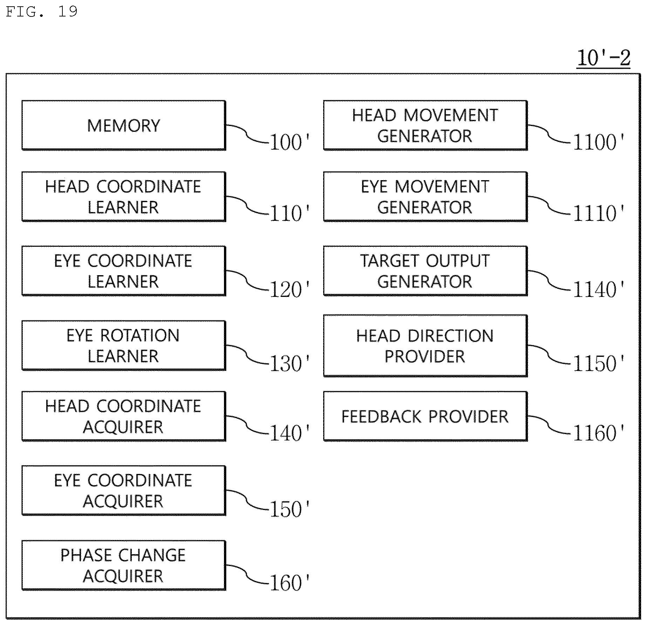

FIG. 19 is a block diagram of a balance function management system for performing a balance function rehabilitation program according to an exemplary embodiment of the present specification.

Referring to FIG. 19, a balance function management system 10'-2 for performing a balance function rehabilitation program according to an exemplary embodiment of the present specification may include the memory 100', the head coordinate learner 110', the eye coordinate learner 120', the eye rotation learner 130', the head coordinate acquirer 140', the eye coordinate acquirer 150', the phase change acquirer 160', the head movement generator 1100', the eye movement generator 1110', a target output generator 1140', a head direction provider 1150', and a feedback provider 1160'. In describing the balance function management system 10'-2, repetitive descriptions of each component will be omitted.

As illustrated in FIG. 11, the target output generator 1140' may output a virtual target 209 to the display device.

The head direction provider 1150' may provide the subject with the information on the rotation direction of the head according to the balance rehabilitation protocol. Since the providing the information on the rotation direction of the head has been described above, a detailed description thereof will be omitted.

The feedback provider 1160' may provide feedback according to the head movement and the eye movement of the subject.

In addition, the eye movement generator 1110' may generate the coordinate information of the gaze point which the subject's gaze faces on the display device using the eye gaze vector. The feedback provider 1160' may compare the coordinate information of the gaze point generated from the eye movement generator 1110' with the coordinate information of the virtual target 209 and may provide feedback. Since the process of providing feedback has been described above, a detailed description thereof will be omitted.

When the subject is captured by the plurality of cameras, the head movement generator 1100' and the eye movement generator 1110' may generate the information related to the reference head movement and the reference eye movement as described above. The feedback provider 1160' may provide the feedback according to the information related to the reference head movement and the reference eye movement.

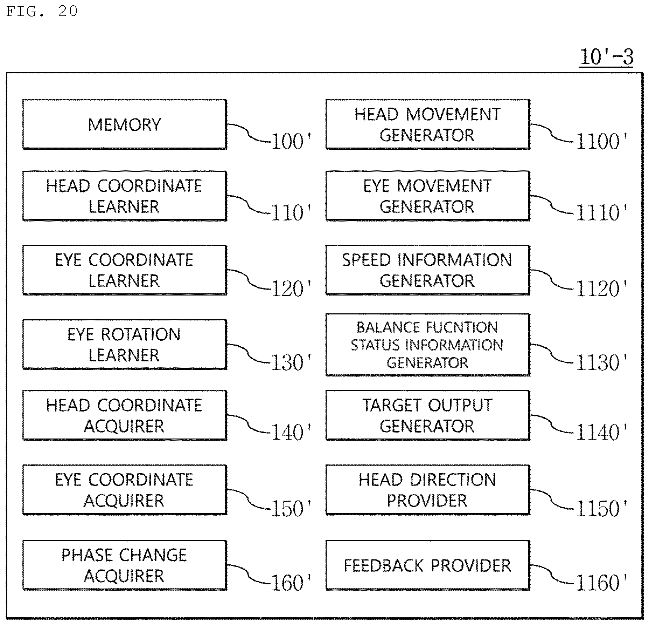

FIG. 20 is a block diagram of a balance function management system for generating information on a balance function status and performing a balance function rehabilitation program according to an exemplary embodiment of the present specification.

Referring to FIG. 20, a balance function management system 10'-3 may include the memory 100', the head coordinate learner 110', the eye coordinate learner 120', the eye rotation learner 130', the head coordinate acquirer 140', the eye coordinate acquirer 150', the phase change acquirer 160', the head movement generator 1100', the eye movement generator 1110', the speed information generator 1120', the balance function status information generator 1130', the target output generator 1140', the head direction provider 1150', and the feedback provider 1160'. The balance function management system 10'-3 may generate a gain to provide a balance function rehabilitation program according to whether the balance function is abnormal.

The head coordinate learners 110 and 110', the eye coordinate learners 120 and 120', the eye rotation learners 130 and 130', the head coordinate acquirers 140 and 140', the eye coordinate acquirers 150 and 150', the phase change acquirers 160 and 160', the head movement generators 1100 and 1100', the eye movement generators 1110 and 1110', the speed information generators 1120 and 1120', the balance function status information generators 1130 and 1130', the target output generators 1140 and 1140', the head direction providers 1150 and 1150', and the feedback providers 1160 and 1160' may include a processor, an application-specific integrated circuit (ASIC), other chipsets, logic circuits, registers, communication modems, data processing units, etc., that are known in the technical field to which the present disclosure pertains to perform calculation and various control logics. In addition, when the above-described control logic is implemented as software, the each component may be implemented as a set of program modules. In this case, the program module may be stored in the memory device and executed by the processor.

Hereinafter, a method of generating information on a balance function status and a balance function rehabilitation method using a balance function management system according to the present specification are disclosed. However, when describing the method of generating information on a balance function status and the balance function rehabilitation method according to the present specification, a repetitive description of each component will be omitted.

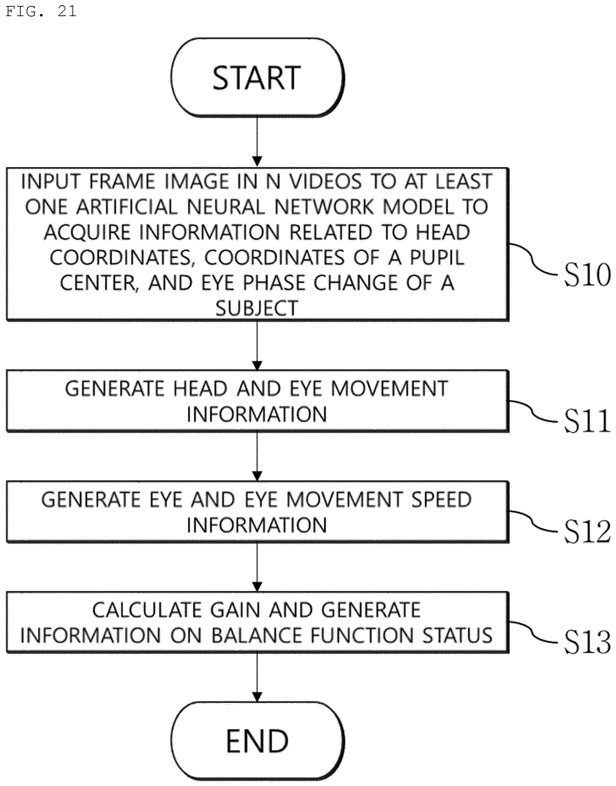

FIG. 21 is a flowchart of a method of generating information on a balance function status according to an exemplary embodiment of the present specification.

Referring to FIG. 21, in step S10, at least one processor may input frame images of n videos to the first ANN model to acquire the information related to the head coordinates. Thereafter, the at least one processor may input the information related to the head coordinates of the n videos to the second ANN model to acquire the information related to the coordinates of a pupil center. Thereafter, the at least one processor may input the information related to the coordinates of a pupil center of the n videos to the third ANN model to acquire the information related to the eye phase changes. Since the learning process of the first to third ANN models has been described above, a repetitive description thereof will be omitted.

In step S11, the at least one processor may generate the head and eye movement information. Alternatively, the at least one processor may further generate the reference head movement information and the reference eye movement information for the m-th video.

In step S12, when the head movement and/or the reference head movement of the subject in the m-th video is greater than or equal to the preset threshold value, the at least one processor may generate the speed information of the head and eye movements for the m-th video.

In step S13, the at least one processor may calculate the gain using the above [Equation 1] and [Equation 2]. The at least one processor may generate the balance function state information using the gain.

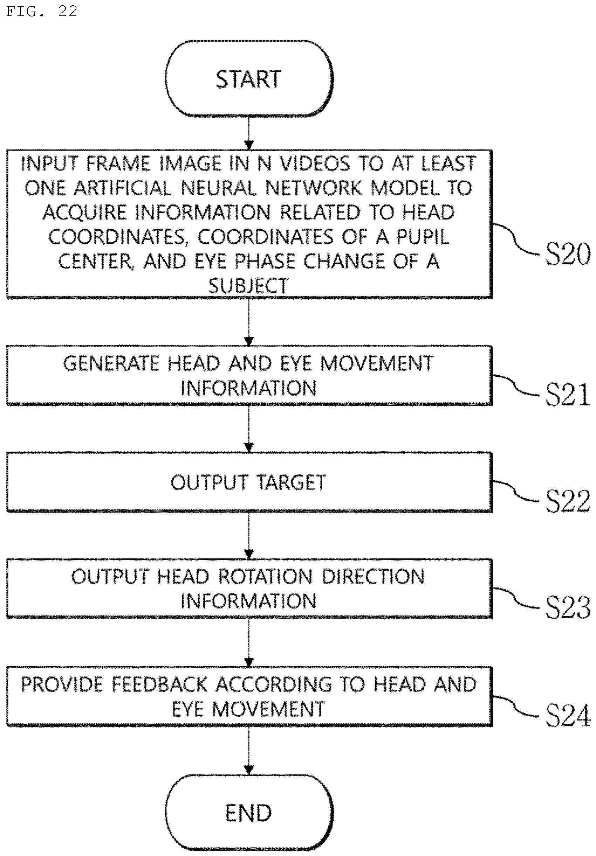

FIG. 22 is a flowchart of a balance function rehabilitation method according to an exemplary embodiment of the present specification. Since step S20 is the same as step S10, a repetitive description thereof will be omitted.

In step S21, the at least one processor may generate the head and eye movement information. The at least one processor may generate the head and eye movements information for the m-th video, respectively. In addition, the at least one processor may further generate the reference head movement information and the reference eye movement information for the m-th video. The at least one processor may generate coordinate information of a gaze point according to a subject's gaze using the reference eye movement information.

In step S22, the at least one processor may output a virtual target to a display. In step S23, the at least one processor may provide information on a rotation direction of a head to a subject.

In step S24, the at least one processor may provide auditory feedback and/or visual feedback to a subject using the head and eye movement information.

**Claims**

1. A balance function management system, comprising:

   at least one processor; and
   a memory that stores instructions executable by the processor and stores at least one artificial neural network model executed on a computing device,
   wherein the at least one processor inputs frame images of n videos of a subject captured by n (natural number) cameras to at least one artificial neural network model to acquire at least one of information related to head coordinates, coordinates of a pupil center, and eye phase changes of the subject according to an order of frame images of an m-th (natural number from 1 to n) video, and
   uses the information to generate information related to head movement and eye movement for generating information on a balance function status or performing a balance function rehabilitation program.

2. The balance function management system according to claim 1, wherein the at least one processor comprising:

   a head coordinate acquirer that executes a first artificial neural network model stored in the memory, and inputs frame images of the m-th video or multi-frame images concatenating frame images of n videos to the first artificial neural network model to acquire the information related to the head coordinates according to the m-th video;
   an eye coordinate acquirer that executes a second artificial neural network model stored in the memory, and inputs the information related to the head coordinates to the second artificial neural network model to acquire information related to coordinates of a pupil center according to the m-th video; and
   a phase change acquirer that executes a third artificial neural network model stored in the memory, and inputs information related to coordinates of a pupil center according to a time sequence of the multi-frame image or the frame image of the m-th video to the third artificial neural network model to acquire information related to the eye phase changes according to the m-th video.

3. The balance function management system according to claim 2, wherein the first artificial neural network model is an artificial neural network model trained by allowing the at least one processor to use, as training data, facial feature points extracted from frame images of at least one video in which a human face is captured or multi-frame images in which frame images of multiple videos in which a human face is captured are concatenated and coordinates of the feature points; wherein the feature point is a feature point positioned within a preset area in the frame images or the multi-frame images.

4. The balance function management system according to claim 2, wherein the second artificial neural network model is an artificial neural network model trained to generate the information related to the coordinates of a pupil center by allowing the at least one processor to use, as training data, eye area images extracted to include an eye from frame images of at least one video in which a human face is captured or multi-frame images in which frame images of multiple videos in which a human face is captured are concatenated.

5. The balance function management system according to claim 4, wherein the at least one processor generates pupil area images in which an area occupied by the pupil and the remaining area have different pixel values in the eye area images, and trains the second artificial neural network model using the pupil area images or an array of pixel values of the pupil area images as the training data.

6. The balance function management system according to claim 4, wherein the at least one processor trains the second artificial neural network model to generate eye feature points and coordinate information of the feature points from the eye area images, and to generate horizontal coordinate values and vertical coordinate values of the pupil center using coordinates of a plurality of preset feature points.

7. The balance function management system according to claim 2, wherein the memory stores data of at least one virtual object, and
the second artificial neural network model is an artificial neural network model trained to generate the information related to the coordinates of a pupil center by allowing the at least one processor to use training data that includes a parameter value that changes at least one of parameters related to head rotation, eye rotation, and camera settings of the virtual object and an image of the virtual object acquired according to the parameter value.

8. The balance function management system according to claim 2, wherein the third artificial neural network model is an artificial neural network model trained to generate an eye rotation value by allowing the at least one processor to use, as training data, information related to eye phase changes generated according to a time sequence of eye area images extracted to include an eye from frame images of at least one video in which a human face is captured or multi-frame images in which frame images of multiple videos in which a human face is captured are concatenated.

9. The balance function management system according to claim 8, wherein the third artificial neural network model is an artificial neural network model trained by allowing the at least one processor to use information comparing pixel values of an area occupied by an iris between eye area images corresponding to each frame image of each video, or to each frame image of each video in the multi-frame images.

10. The balance function management system according to claim 8, wherein the at least one processor calculates a size of an area occupied by a pupil in the eye area images and adjusts a size of a target eye area image using the size of the area occupied by the pupil in a preceding eye area image.

11. The balance function management system according to claim 1, wherein the at least one processor comprising:

a head movement generator that generates information related to head movement in the m-th (natural number from 1 to n) video using the information related to the head coordinates generated from the at least one artificial neural network model;
an eye movement generator that generates information related to eye movement in the m-th video using the information related to the coordinates of the pupil center or the eye phase changes generated from the at least one artificial neural network model;
a speed information generator that generates information related to head and eye movement speeds in the m-th video using the information related to the head movement and the eye movement; and
a balance function status information generator that generates information on a balance function status of the subject using the information related to the head and eye movement speeds.

12. The balance function management system according to claim 11, wherein the speed information generator generates the information related to the head movement speed and the eye movement speed within a preset time based on a time when the head movement becomes greater than or equal to a preset threshold value.

13. The balance function management system according to claim 11, wherein the balance function status information generator calculates a gain using a time value at which the head movement speed is maximum and a time value at which the eye movement speed is maximum.

14. The balance function management system according to claim 11, wherein, when n is greater than or equal to 2,

the head movement generator further generates reference head movement information by calculating statistical values of the information related to the head coordinates according to the m-th video, and

the eye movement generator further generates reference eye movement information by calculating statistical values of the information related to the coordinates of the pupil center and the eye phase changes according to the m-th video.

15. The balance function management system according to claim 1, wherein the at least one processor comprising:

a head movement generator that generates the information related to head movement in the m-th (natural number from 1 to n) using the information related to the head coordinates generated from the at least one artificial neural network model;

an eye movement generator that generates the eye movement information in the m-th video using the information related to the coordinates of a pupil center or the eye phase changes acquired from the at least one artificial neural network model;

a target output generator that outputs a virtual target to a display device;

a head direction provider that provides direction information on the head movement to the subject; and

a feedback provider that provides feedback according to the head movement and the eye movement of the subject.

FIG. 1

FIG. 2

FIG. 3

<u>10</u>

| | |
|---|---|
| MEMORY | 100 |
| HEAD COORDINATE LEARNER | 110 |
| EYE COORDINATE LEARNER | 120 |
| EYE ROTATION LEARNER | 130 |
| HEAD COORDINATE ACQUIRER | 140 |
| EYE COORDINATE ACQUIRER | 150 |
| PHASE CHANGE ACQUIRER | 160 |

FIG. 4

FIG. 5

FIG. 6

FIG. 7

| Parameters | | | |
|---|---|---|---|
| | Roll | Pitch | Yaw |
| Neck | 0.00 | 0.00 | 0.00 |
| Jaw | 0.00 | 0.00 | 0.00 |
| Eyes | 0.00 | 0.00 | 0.00 |

Render
Number of Points: 80520
Scale: 1.00
FoV (Vertical): 18 deg

| Parameters | | | |
|---|---|---|---|
| | Roll | Pitch | Yaw |
| Neck | 0.00 | 0.00 | 0.00 |
| Jaw | 0.00 | 0.00 | 0.00 |
| Eyes | 0.07 | -0.02 | -0.12 |

Render
Number of Points: 80520
Scale: 1.00
FoV (Vertical): 18 deg

FIG. 8

<u>10-1</u>

FIG. 9

EP 4 693 333 A1

FIG. 10

<u>10-2</u>

| | |
|---|---|
| MEMORY — 100 | HEAD MOVEMENT GENERATOR —1100 |
| HEAD COORDINATE LEARNER — 110 | EYE MOVEMENT GENERATOR —1110 |
| EYE COORDINATE LEARNER — 120 | TARGET OUTPUT GENERATOR —1140 |
| EYE ROTATION LEARNER — 130 | HEAD DIRECTION PROVIDER —1150 |
| HEAD COORDINATE ACQUIRER — 140 | FEEDBACK PROVIDER —1160 |
| EYE COORDINATE ACQUIRER — 150 | |
| PHASE CHANGE ACQUIRER — 160 | |

FIG. 11

FIG. 12

10-3

| MEMORY | 100 |
| --- | --- |

| HEAD COORDINATE LEARNER | 110 |
| --- | --- |

| EYE COORDINATE LEARNER | 120 |
| --- | --- |

| EYE ROTATION LEARNER | 130 |
| --- | --- |

| HEAD COORDINATE ACQUIRER | 140 |
| --- | --- |

| EYE COORDINATE ACQUIRER | 150 |
| --- | --- |

| PHASE CHANGE ACQUIRER | 160 |
| --- | --- |

| HEAD MOVEMENT GENERATOR | 1100 |
| --- | --- |

| EYE MOVEMENT GENERATOR | 1110 |
| --- | --- |

| SPEED INFORMATION GENERATOR | 1120 |
| --- | --- |

| BALANCE FUCNTION STATUS INFORMATION GENERATOR | 1130 |
| --- | --- |

| TARGET OUTPUT GENERATOR | 1140 |
| --- | --- |

| HEAD DIRECTION PROVIDER | 1150 |
| --- | --- |

| FEEDBACK PROVIDER | 1160 |
| --- | --- |

FIG. 13

<u>10'</u>

| |
|---|
| MEMORY ~100' |
| HEAD COORDINATE LEARNER ~110' |
| EYE COORDINATE LEARNER ~120' |
| EYE ROTATION LEARNER ~130' |
| HEAD COORDINATE ACQUIRER ~140' |
| EYE COORDINATE ACQUIRER ~150' |
| PHASE CHANGE ACQUIRER ~160' |

FIG. 14

FIG. 15

FIG. 16

FIG. 17

FIG. 18

<u>10'-1</u>

| | |
|---|---|
| MEMORY ~100' | HEAD MOVEMENT GENERATOR ~1100' |
| HEAD COORDINATE LEARNER ~110' | EYE MOVEMENT GENERATOR ~1110' |
| EYE COORDINATE LEARNER ~120' | SPEED INFORMATION GENERATOR ~1120' |
| EYE ROTATION LEARNER ~130' | BALANCE FUCNTION STATUS INFORMATION GENERATOR ~1130' |
| HEAD COORDINATE ACQUIRER ~140' | |
| EYE COORDINATE ACQUIRER ~150' | |
| PHASE CHANGE ACQUIRER ~160' | |

EP 4 693 333 A1

FIG. 19

40

FIG. 20

10'-3

| | |
|---|---|
| MEMORY ~100' | HEAD MOVEMENT GENERATOR ~1100' |
| HEAD COORDINATE LEARNER ~110' | EYE MOVEMENT GENERATOR ~1110' |
| EYE COORDINATE LEARNER ~120' | SPEED INFORMATION GENERATOR ~1120' |
| EYE ROTATION LEARNER ~130' | BALANCE FUCNTION STATUS INFORMATION GENERATOR ~1130' |
| HEAD COORDINATE ACQUIRER ~140' | TARGET OUTPUT GENERATOR ~1140' |
| EYE COORDINATE ACQUIRER ~150' | HEAD DIRECTION PROVIDER ~1150' |
| PHASE CHANGE ACQUIRER ~160' | FEEDBACK PROVIDER ~1160' |

FIG. 21

```
                    ┌─────────────┐
                    │    START    │
                    └──────┬──────┘
                           │
                           ▼
   ┌──────────────────────────────────────────────┐
   │ INPUT FRAME IMAGE IN N VIDEOS TO AT LEAST     │
   │ ONE ARTIFICIAL NEURAL NETWORK MODEL TO        │
   │ ACQUIRE INFORMATION RELATED TO HEAD           │ ─── S10
   │ COORDINATES, COORDINATES OF A PUPIL           │
   │ CENTER, AND EYE PHASE CHANGE OF A             │
   │ SUBJECT                                        │
   └──────────────────────┬───────────────────────┘
                           │
                           ▼
   ┌──────────────────────────────────────────────┐
   │ GENERATE HEAD AND EYE MOVEMENT                │ ─── S11
   │ INFORMATION                                    │
   └──────────────────────┬───────────────────────┘
                           │
                           ▼
   ┌──────────────────────────────────────────────┐
   │ GENERATE EYE AND EYE MOVEMENT SPEED           │ ─── S12
   │ INFORMATION                                    │
   └──────────────────────┬───────────────────────┘
                           │
                           ▼
   ┌──────────────────────────────────────────────┐
   │ CALCULATE GAIN AND GENERATE                   │ ─── S13
   │ INFORMATION ON BALANCE FUNCTION STATUS        │
   └──────────────────────┬───────────────────────┘
                           │
                           ▼
                    ┌─────────────┐
                    │     END     │
                    └─────────────┘
```

FIG. 22

```
                    ┌──────────────┐
                    │    START     │
                    └──────┬───────┘
                           │
                           ▼
┌──────────────────────────────────────────────┐
│  INPUT FRAME IMAGE IN N VIDEOS TO AT LEAST     │
│  ONE ARTIFICIAL NEURAL NETWORK MODEL TO        │ ─── S20
│  ACQUIRE INFORMATION RELATED TO HEAD           │
│  COORDINATES, COORDINATES OF A PUPIL           │
│  CENTER, AND EYE PHASE CHANGE OF A             │
│  SUBJECT                                       │
└───────────────────────┬────────────────────────┘
                        │
                        ▼
┌──────────────────────────────────────────────┐
│      GENERATE HEAD AND EYE MOVEMENT            │ ─── S21
│              INFORMATION                       │
└───────────────────────┬────────────────────────┘
                        │
                        ▼
┌──────────────────────────────────────────────┐
│               OUTPUT TARGET                    │ ─── S22
└───────────────────────┬────────────────────────┘
                        │
                        ▼
┌──────────────────────────────────────────────┐
│      OUTPUT HEAD ROTATION DIRECTION            │ ─── S23
│              INFORMATION                       │
└───────────────────────┬────────────────────────┘
                        │
                        ▼
┌──────────────────────────────────────────────┐
│  PROVIDE FEEDBACK ACCORDING TO HEAD AND        │ ─── S24
│             EYE MOVEMENT                       │
└───────────────────────┬────────────────────────┘
                        │
                        ▼
                 ┌──────────────┐
                 │     END      │
                 └──────────────┘
```

Europäisches
Patentamt

European
Patent Office

Office européen
des brevets

# EUROPEAN SEARCH REPORT

**Application Number**

EP 25 16 8966

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/007849 A1 (KRUEGER WESLEY W O [US]) 14 January 2016 (2016-01-14) * The whole document, in particular: Paragraphs [0001], [0017], [0030], [0076], [0077], [0080], [0105], [0130], [0168]; Figure 2. * ----- | 1-15 | INV. G16H50/20 G16H20/30 G16H30/40 G16H50/70 A61B5/00 |
| X | US 2020/397288 A1 (ZIDAN AWSS [US] ET AL) 24 December 2020 (2020-12-24) * The whole document, in particular: Claims 1, 2; Paragraphs [0078] - [0193], [0220] - [0224], [0247] - [0249], [0266] - [0274]. * ----- | 1-15 | ADD. A61B3/113 A61B5/11 |
| A | WO 2024/132135 A1 (PUPIL LABS GMBH [DE]) 27 June 2024 (2024-06-27) * The whole document, in particular: Claims 1 - 8; Paragraphs [001] - [006], [00125] [00137]. * ----- | 1-15 | |

**TECHNICAL FIELDS SEARCHED (IPC)**

G16H
A61B

The present search report has been drawn up for all claims

| Place of search | Date of completion of the search | Examiner |
|---|---|---|
| Munich | 11 September 2025 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 16 8966

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2025

| Patent document cited in search report | Publication date | Patent family member(s) | | Publication date |
|---|---|---|---|---|
| US 2016007849 A1 | 14-01-2016 | NONE | | |
| US 2020397288 A1 | 24-12-2020 | NONE | | |
| WO 2024132135 A1 | 27-06-2024 | CN | 120380439 A | 25-07-2025 |
| | | WO | 2024132135 A1 | 27-06-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240104114
- KR 1020240104115

- KR 1020040107677